# FASCICULE DE BREVET EUROPEEN

(11) **EP 2 723 719 B1**
(45) Date de publication et mention de la délivrance du brevet: **30.11.2016**
(21) Numéro de dépôt: 12730905.2
(22) Date de dépôt: 22.06.2012
(51) Int. Cl.: C07D 213/74, C07D 241/20, C07D 295/088, C07D 295/096, C07D 277/42, C07D 495/04, C07D 295/155, C07D 295/26, C07D 241/04, C07D 307/33, C07D 295/192, C07D 295/205, A61K 31/495, A61P 3/10

(54) **DÉRIVÉS DE PIPÉRAZINE, LEURS PROCÉDÉS DE PRÉPARATION ET LEURS UTILISATIONS DANS LE TRAITEMENT DE L'INSULINORÉSISTANCE**
PIPERAZIN-DERIVATE, DEREN HERSTELLUNGSVERFAHREN UND DEREN VERWENDUNGEN IN DER BEHANDLUNG VON INSULINORESISTENZ
PIPERAZINE DERIVATIVES, THEIR PREPARATION PROCESSES AND THEIR USES IN THE TREATMENT OF INSULINORESISTANCE

(30) Priorité: 23.06.2011 FR 1155547
(43) Date de publication de la demande: 30.04.2014
(73) Titulaire: Metabolys, 69372 Lyon Cedex 08 (FR)
(72) Inventeur: MOINET, Gérard, F-91400 Orsay (FR); BAVEREL, Gabriel, F-69450 Saint Cyr Au Mont D'or (FR); NAZARET, Rémi, F-01000 Bourg En Bresse (FR); FERRIER, Bernard, F-69007 Lyon (FR)
(74) Mandataire: Lavoix
(86) Numéro de dépôt international: PCT/EP2012/062154
(87) Numéro de publication internationale: WO 2012/175715

(56) Documents cités:
- WO-A1-00/06558
- RANJU BANSAL ET AL: "Synthesis and vasodilatory activity of some amide derivatives of 6-(4-carboxymethyloxyphenyl)-4,5-dihydro-3 (2H)-pyridazinone", EUROPEAN JOURNAL OF MEDICINAL CHEMISTRY, vol. 44, no. 11, 2009, pages 4441-4447, XP055010265,

## Description

La présente invention concerne de nouveaux composés utiles notamment dans le traitement de pathologies associées au syndrome d'insulinorésistance (ou syndrome X), notamment dans le traitement du diabète de type (II).

On observe depuis quelques années une augmentation importante des cas de diabète dans le monde. Actuellement, on compte environ 250 millions de diabétiques dans le monde et les prévisions pour 2030 sont de l'ordre de plus de 400 millions de diabétiques. Le diabète de type (I) (destruction des cellules produisant de l'insuline) est principalement traité par l'injection d'insuline. Le diabète de type (II), qui est le plus répandu (90% des cas de diabète), se caractérise par une résistance des tissus face à l'insuline et nécessite un traitement particulier.

De nombreux composés ont été proposés pour le traitement du diabète, notamment du diabète de type (II). On connaît notamment de FR2781797 et WO00/06558 des dérivés de pipérazine. On connaît également de WO02/100341 des dérivés phénylés.

Actuellement, dans environ 40% des cas, le traitement n'est pas efficace et le seuil de glycémie à jeun souhaité de 1,26 g/litre de sang n'est pas atteint.

Il est donc nécessaire de proposer de nouveaux composés permettant un traitement plus efficace des pathologies associées au syndrome d'insulinorésistance.

Un objectif de la présente invention est donc de fournir des composés efficaces dans le traitement des pathologies associées au syndrome d'insulinorésistance.

Un autre objectif de la présente invention est de fournir un procédé de préparation de ces composés.

Un autre objectif encore de la présente invention est de fournir un moyen de traitement de pathologies associées au syndrome d'insulinorésistance, notamment diabète de type (II). Un objectif de la présente invention est de proposer des composés permettant notamment d'inhiber la néoglucogenèse.

D'autres objectifs encore apparaîtront à la lecture de la description de l'invention qui suit.

La présente invention concerne un composé de formule (I) 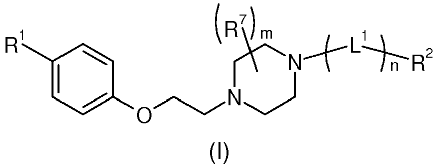 dans laquelle n représente 0 ou 1, leurs énantiomères, diastéréoisomères et leurs sels d'addition avec des bases ou des acides pharmaceutiquement acceptables.

L'invention concerne également un composé de formule (I) dans laquelle n=0, correspondant ainsi au composé de formule (Ia) suivant, leurs énantiomères, diastéréoisomères et leurs sels d'addition avec des bases ou des acides pharmaceutiquement acceptables :

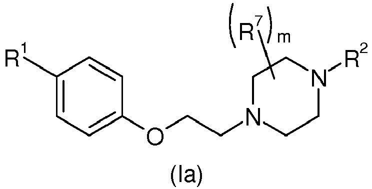

L'invention concerne également un composé de formule (I) dans laquelle n=1, correspondant ainsi au composé de formule (Ib) suivant leurs énantiomères, diastéréoisomères et leurs sels d'addition avec des bases ou des acides pharmaceutiquement acceptables :

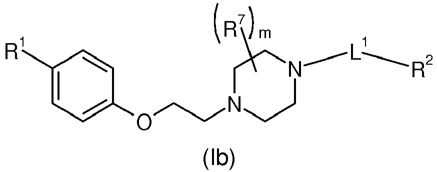

Dans les composés de formule (I), (Ia) et (Ib) selon l'invention :
▪ R¹ représente :
   - un groupe -C(O)CR³R⁴CR⁵R⁶C(O)OH ; ou
   - un groupe 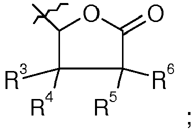
▪ m représente 0 ou 1 ;
▪ L¹ représente un groupe -C(O)- ; -C(O)O- ou -S(O)₂- ;
▪ R² représente :
   - un groupe phényle substitué ou non substitué ;
   - un groupe hétérocycle aromatique en 5, 6 ou 7 membres pouvant comprendre 1, 2 ou 3 hétéroatomes, identiques ou différents, choisis parmi l'azote, l'oxygène ou le soufre ;
   - un groupe polyhétérocycle aromatique en 9 ou 10 membres pouvant comprendre 1, 2 ou 3 hétéroatomes, identiques ou différents, choisis parmi l'azote, l'oxygène ou le soufre ;
   - un groupe -L²- -phényle;
   - un groupe hydrocarboné, linéaire ou ramifié, en C₁ à C₅, de préférence C₁ à C₄, de préférence alkyle, linéaire ou ramifié, en C₁ à C₅, de préférence C₁ à C₄ ;
▪ L² étant un alkyle, linéaire ou ramifié en C₁ à C₅, de préférence en C₁ à C₄ ;
▪ R³, R⁴, R⁵ et R⁶ représentent un atome d'hydrogène
▪ R⁷ représente un alkyle, linéaire ou ramifié, en C₁ à C₅, de préférence C₁ à C₄ ; par exemple méthyle, éthyle, propyle, iso-propyle, butyle, ter-butyle ;

Le groupe phényle est non substitué ou substitué par un ou plusieurs substituants, identiques ou différents, choisi parmi :
- un groupe alkoxy en C₁ à C₆, linéaire ou ramifié, par exemple méthoxy, éthoxy, propoxy, iso-propoxy, butoxy, ter-butoxy ;
- un atome d'halogène ;
- un groupe hydrocarboné, linéaire ou ramifié, de préférence alkyle, en C₁ à C₅, de préférence en C₁ à C₄, par exemple méthyle, éthyle, propyle, butyle, iso-propyle, ter-butyle ;
- un groupe hydrocarboné, linéaire ou ramifié, de préférence alkyle, en C₁ à C₅, de préférence en C₁ à C₄, substitué par un ou plusieurs atomes d'halogène ;
- un groupe cyano (-CN) ; ou
- un groupe alkyle sulfonyle (-S(O)₂-alkyle), dans lequel l'alkyle est linéaire ou ramifié, en C₁ à C₅, de préférence en C₁ à C₄, par exemple méthyle, éthyle, propyle, butyle, iso-propyle, ter-butyle

De façon préférée, dans le composé de formule (I), (la) et (Ib), R¹ représente un groupe -C(O)CR³R⁴CR⁵R⁶C(O)OH.

De façon préférée, dans le composé de formule (I), (Ia) et (Ib), m représente 0.

De façon préférée, dans le composé de formule (I), (Ia) et (Ib), R² représente :
- un phényl, non substitué ou substitué par un ou plusieurs substituants, identiques ou différents, choisi parmi :
   - un groupe alkoxy en C₁ à C₆, de préférence en C₁ à C₃, linéaire ou ramifié, par exemple méthoxy, éthoxy, propoxy, iso-propoxy, butoxy, ter-butoxy ;
   - un atome d'halogène, par exemple fluor, chlore, brome ;
   - un groupe alkyle en C₁ à C₅, de préférence en C₁ à C₄, linéaire ou ramifié, par exemple méthyle, éthyle, propyle, butyle, iso-propyle, ter-butyle ;
   - un groupe alkyle en C₁ à C₅, de préférence en C₁ à C₄, linéaire ou ramifié, substitué par un ou plusieurs atomes d'halogène, par exemple trifluorométhyl ;
   - un groupe cyano (-CN) ; ou
   - un groupe alkylsulfonyle (-S(O)₂-alkyl), dans lequel l'alkyle est linéaire ou ramifié, en C₁ à C₅, de préférence en C₁ à C₄, par exemple méthyle, éthyle, propyle, butyle, iso-propyle, ter-butyle, par exemple méthane sulfonyle (-S(O)₂CH₃) ;
   de préférence le ou les substituants sont en position ortho ou para sur le phényl ;
- un hétéroaryl monocyclique ou polycyclique choisi parmi 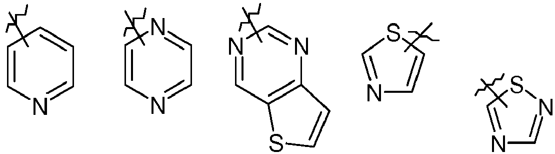 de préférence l'hétéroaryl mono ou polycyclique est choisi parmi 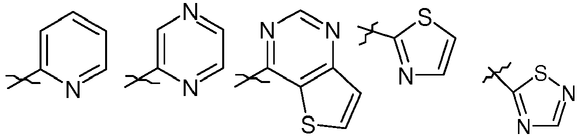 2
- un groupe - L²-phényle, L² étant un alkyle, linéaire ou ramifié en C₁ à C₅, de préférence en C₁ à C₄, de préférence méthyle, éthyle, propyle, butyle, iso-propyle, butyle, ter-butyle, par exemple -CH₂- ; ou
- un groupe alkyle, linéaire ou ramifié, en C₁ à C₅, de préférence en C₁ à C₄, par exemple méthyle, éthyle, propyle, butyle, iso-propyle, ter-butyle.

Par polyhétérocycle selon l'invention on entend des hétérocycles polycycliques, notamment comprenant deux cycles fusionnés.

De préférence, et sauf indications contraires, dans les composés de formule (I), (la) et (Ib) selon l'invention les polyhétérocycles comprennent 9 ou 10 membres, et peuvent comprendre 1, 2 ou 3 hétéroatomes, identiques ou différents, choisis parmi l'azote, l'oxygène ou le soufre, et représentent notamment

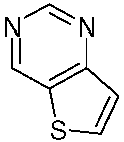

De préférence, et sauf indications contraires, dans les composés de formule (I), (la) et (Ib) selon l'invention les hétérocycles comprennent 5 ou 6 membres, et peuvent comprendre 1, 2 ou 3 hétéroatomes, identiques ou différents, choisis parmi l'azote, l'oxygène ou le soufre, et sont par exemple choisis parmi

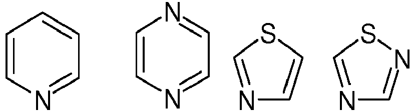

L'invention se rapporte également aux solvates des composés de formules (I), (Ia) et (Ib). Les composés de formules (I), (Ia) et (Ib) possèdent une fonction carboxylique et peuvent être salifiés. Ils peuvent alors se présenter sous la forme de sels d'addition avec des bases organiques ou minérales. Les sels d'addition avec des bases sont par exemple des sels pharmaceutiquement acceptables tels que les sels de sodium, les sels de potassium, les sels de calcium, lesquels sont obtenus en utilisant des hydroxydes de métaux alcalins et alcalino-terreux correspondants comme bases. Comme autre type de sels d'addition avec des bases pharmaceutiquement acceptables, on peut citer les sels avec des amines et notamment la glucamine, le N-méthylglucamine, le N,N-diméthylglucamine, l'éthanolamine, la morpholine, la N-méthylmorpholine ou la lysine.

Les composés de formules (I), (Ia) et (Ib) peuvent également être salifiés avec des acides minéraux ou organiques et de préférence des acides pharmaceutiquement acceptables tels que les acides chlorhydrique, phosphorique, fumarique, citrique, oxalique, sulfurique, ascorbique, tartrique, maléique, mandélique, méthanesulfonique, lactobionique, gluconique, glucarique, succinique, sulfonique ou hydroxypropanesulfonique.

La présente invention concerne également un premier procédé (P1) de préparation des composés de formule (I) comprenant :
(a) la protection de la fonction acide (portée par R¹) d'un composé de formule (II) 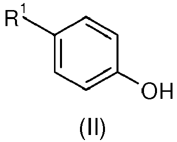 dans laquelle R¹ représente -C(O)CR³R⁴CR⁵R⁶C(O)OH ;
(b) la réaction du composé obtenu à l'étape (a) avec un composé de formule R⁹-(CH₂)₂-R⁸ dans laquelle R⁸ et R⁹, identiques ou différents représentent un groupe partant, de préférence R⁹ est meilleur nucléofuge que R⁸ ;
(c) la réaction du composé obtenu à l'étape (b) avec un composé de formule (III) 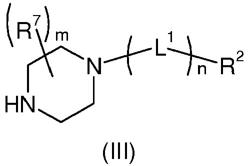 dans laquelle L¹, n, m, R² et R⁷ ont les définitions données pour la formule (I) ;
(d) déprotection du composé obtenu à l'étape (c).

Les produits de départ sont commerciaux ou peuvent être facilement préparés par l'homme du métier sur la base de ses connaissances générales en chimie organique.

Les composés pour lesquels R¹ représente -C(OH)(H)CR³R⁴CR⁵R⁶C(O)OH peuvent être obtenus par réduction des composés pour lesquels R¹ représente -C(O)CR³R⁴CR⁵R⁶C(O)OH. Cette réduction peut être mise en oeuvre par exemple en présence de NaBH₄ dans l'éthanol. Cette réduction peut, par exemple, intervenir entre les étapes (b) et (c).

Les composés pour lesquels R¹ représente 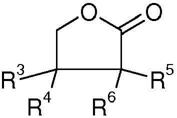 peuvent être obtenus par :
- lactonisation des composés pour lesquels R¹ représente -C(OH)(H)CR³R⁴CR⁵R⁶C(O)OH. Cette lactonisation aura par exemple lieu avant l'étape (c) ; ou
- lactonisation des composés pour lesquels R¹ représente -C(O)CR³R⁴CR⁵R⁶C(O)OH. Cette lactonisation aura par exemple lieu après l'étape (d).
Ces lactonisations peuvent être réalisées par toute méthode connue de l'homme du métier, elles peuvent par exemple être réalisées en présence de CF₃CO₂H dans le dichlorométhane.

L'étape (a), correspondant à la protection de la fonction acide, peut se faire de toute manière connue par l'homme du métier, pourvue que la protection soit sélective de la fonction acide par rapport à la fonction alcool. Parmi les groupes protecteurs de la fonction carboxylique, ceux généralement décrits dans Protective Groups in Organic Synthesis, Greene T.W. et Wuts P.G.M, ed. John Wiley and Sons, 1991 et dans Protective Groups, Kocienski P.J., 1994, Georg Thieme Verlag, peuvent convenir. A titre d'exemple on peut envisager la protection de la fonction carboxylique sous la forme d'ester (alkyle en C₁-C₆, par exemple méthyle). Par exemple l'étape (a) peut se faire par réaction du composé de formule (I) avec le méthanol en présence d'un acide, notamment l'acide sulfurique.
L'étape (b) est de préférence mise en oeuvre dans un solvant polaire aprotique tel que l'acétonitrile, le diméthylformamide (DMF), l'acétone, le diméthylsulfoxide et les hydrocarbures halogénés tels que le dichlorométhane ou le dichloroéthane, à une température adaptée, notamment comprise entre 15 et 80°C, de préférence entre 15 et 35°C. Un solvant préféré est notamment l'acétonitrile. De façon avantageuse, cette étape a lieu en présence d'une base telle que le carbonate de potassium. L'homme du métier sait qu'un groupe partant est d'autant plus labile que l'espèce anionique correspondante est stable. Ainsi, R⁹ est plus nucléofuge que R⁸ correspond au fait que R⁹- est plus stable que R⁸-. Les groupes partants R⁸ et R⁹, identiques ou différents, sont choisis parmi les atomes d'halogène, de préférence chlore et brome ; les groupes (C₆-C₁₀)arylsulfonyloxy, le groupe aryle étant éventuellement substitué par un ou plusieurs groupes alkyle en C₁ à C₆ ; les groupes (C₁-C₆)alkylsulfonyloxy dans lequel le groupe alkyle est éventuellement substitué par un ou plusieurs atomes d'halogène. De préférence R⁸ représente le chlore et R⁹ représente le brome.

L'étape (c) est une étape de substitution nucléophile pour laquelle les conditions opératoires pourront être facilement déterminées par l'homme du métier. Avantageusement, la réaction est mise en oeuvre dans un solvant polaire aprotique en présence d'une base. Des exemples de solvant appropriés sont l'acétonitrile, le diméthylformamide, l'acétone, le diméthylsulfoxide et les hydrocarbures halogénés tels que le dichlorométhane ou le dichloroéthane. A titre de base on peut citer les carbonates de métal alcalin ou alcalino-terreux, par exemple le carbonate de potassium. De façon avantageuse, la réaction de l'étape (c) est conduite à une température de 50 à 120°C, par exemple au reflux du solvant en présence d'un iodure de métal alcalin tel que l'iodure de potassium. La quantité d'iodure alcalin peut être variable et dépend essentiellement de la nature des réactifs, de la nature du solvant et de la température réactionnelle. La réaction est généralement stoechiométrique, il est néanmoins possible de travailler avec un léger excès de l'un ou de l'autre des réactifs.
L'étape de déprotection (d) peut être toute déprotection connue de l'homme du métier et compatible avec la protection mise en oeuvre à l'étape (a) permettant de récupérer la fonction acide. Par exemple, il peut s'agir d'une saponification en milieu basique, acide ou catalytique (Pd/C).

Pour la préparation du composé de formule (la), le même procédé (P1) s'applique avec le composé de formule (IIIa) 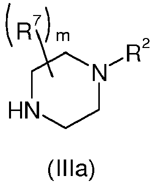 dans laquelle m, R² et R⁷ sont tels que définis pour la formule (Ia).
Les produits de départ sont commerciaux ou peuvent être facilement préparés par l'homme du métier sur la base de ses connaissances générales en chimie organique.

Pour la préparation du composé de formule (Ib), le même procédé (P1) s'applique avec le composé de formule (IIIb) 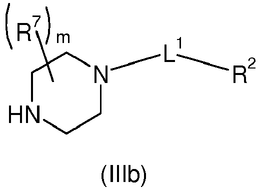 dans laquelle L¹, n, m, R² et R⁷ ont les définitions données pour la formule (Ib).

Les produits de départ sont commerciaux ou peuvent être facilement préparés par l'homme du métier sur la base de ses connaissances générales en chimie organique.

L'invention concerne également un second procédé (P2) de préparation d'un composé de formule (I) comprenant :
(i) la réaction d'un composé de formule (III) avec un composé de formule R⁹-(CH₂)₂-R⁸, dans laquelle R⁸ et R⁹ sont tels que définis dans le procédé (P1);
(ii) la protection de la fonction acide d'un composé de formule (II) ;
(iii) la réaction entre le composé obtenu à l'étape (i) et le composé obtenu à l'étape (ii) ;
(iv) la déprotection du composé obtenu à l'étape (iii).

Les produits de départ sont commerciaux ou peuvent être facilement préparés par l'homme du métier sur la base de ses connaissances générales en chimie organique.

Les composés pour lesquels R¹ représente -C(OH)(H)CR³R⁴CR⁵R⁶C(O)OH peuvent être obtenus par réduction des composés pour lesquels R¹ représente -C(O)CR³R⁴CR⁵R⁶C(O)OH. Cette réduction peut être mise en oeuvre par exemple en présence de NaBH₄ dans l'éthanol. Cette réduction peut, par exemple, intervenir entre les étapes (ii) et (iii).

Les composés pour lesquels R¹ représente 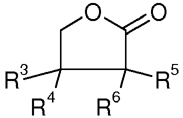 peuvent être obtenus par :
- lactonisation des composés pour lesquels R¹ représente -C(OH)(H)CR³R⁴CR⁵R⁶C(O)OH. Cette lactonisation aura par exemple lieu avant l'étape (iii) ; ou
- lactonisation des composés pour lesquels R¹ représente -C(O)CR³R⁴CR⁵R⁶C(O)OH. Cette lactonisation aura par exemple lieu après l'étape (iv). Ces lactonisations peuvent être réalisées par toute méthode connue de l'homme du métier, elles peuvent par exemple être réalisées en présence de CF₃CO₂H dans le dichlorométhane.

Il existe généralement un équilibre entre les formes pour lesquels R¹ représente -C(OH)(H)CR³R⁴CR⁵R⁶C(O)OH et les formes pour lesquels R¹ représente

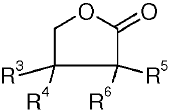

L'étape (i) est de préférence mise en oeuvre dans un solvant polaire aprotique tel que l'acétonitrile, le diméthylformamide (DMF), l'acétone, le diméthylsulfoxide et les hydrocarbures halogénés tels que le dichlorométhane ou le dichloroéthane, à une température adaptée, notamment comprise entre 15 et 80°C, de préférence entre 15 et 35°C. Un solvant préféré est notamment l'acétonitrile. De façon avantageuse cette étape a lieu en présence d'une base telle que le carbonate de potassium.
L'étape (ii), correspondant à la protection de la fonction acide, peut se faire de toute manière connue par l'homme du métier, pourvue que la protection soit sélective de la fonction acide par rapport à la fonction alcool. Parmi les groupes protecteurs de la fonction carboxylique, ceux généralement décrits dans Protective Groups in Organic Synthesis, Greene T.W. et Wuts P.G.M, ed. John Wiley and Sons, 1991 et dans Protective Groups, Kocienski P.J., 1994, Georg Thieme Verlag, peuvent convenir. A titre d'exemple on peut envisager la protection de la fonction carboxylique sous la forme d'ester (alkyle en C₁-C₆, par exemple méthyle). Par exemple l'étape (a) peut se faire par réaction du composé de formule (I) avec le méthanol en présence d'un acide, notamment l'acide sulfurique.
L'étape (iii) est une étape de substitution nucléophile pour laquelle les conditions opératoires pourront être facilement déterminées par l'homme du métier. Avantageusement, la réaction est mise en oeuvre dans un solvant polaire aprotique en présence d'une base. Des exemples de solvant appropriés sont l'acétonitrile, le diméthylformamide, l'acétone, le diméthylsulfoxide et les hydrocarbures halogénés tels que le dichlorométhane ou le dichloroéthane. A titre de base on peut citer les carbonates de métal alcalin ou alcalino-terreux, par exemple le carbonate de potassium. De façon avantageuse, la réaction de l'étape (c) est conduite à une température de 50 à 120°C, par exemple au reflux du solvant en présence d'un iodure de métal alcalin tel que l'iodure de potassium. La quantité d'iodure alcalin peut être variable et dépend essentiellement de la nature des réactifs, de la nature du solvant et de la température réactionnelle. La réaction est généralement stoechiométrique, il est néanmoins possible de travailler avec un léger excès de l'un ou de l'autre des réactifs.
L'étape de déprotection (iv) peut être toute déprotection connue de l'homme du métier et compatible avec la protection mise en oeuvre à l'étape (ii) permettant de récupérer la fonction acide. Par exemple, il peut s'agir d'une saponification en milieu basique, acide ou catalytique (Pd/C).

Pour la préparation du composé de formule (Ia), le même procédé (P2) s'applique avec le composé de formule (IIIa) 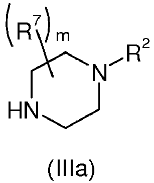 dans laquelle m, R² et R⁷ sont tels que définis pour la formule (Ia).
Les produits de départ sont commerciaux ou peuvent être facilement préparés par l'homme du métier sur la base de ses connaissances générales en chimie organique.

Pour la préparation du composé de formule (Ib), le même procédé (P2) s'applique avec le composé de formule (IIIb) 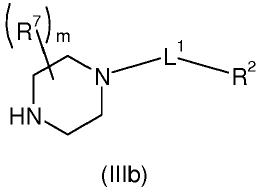 dans laquelle L¹, n, m, R² et R⁷ ont les définitions données pour Ia formule (Ib).
Les produits de départ sont commerciaux ou peuvent être facilement préparés par l'homme du métier sur la base de ses connaissances générales en chimie organique.

Les composés de la présente invention sont utiles comme médicament. Ils sont notamment utiles pour le traitement de pathologies associées au syndrome d'insulino-résistance (ou syndrome X), notamment du diabète de type II.
L'invention concerne également l'utilisation des composés selon l'invention en combinaison avec d'autres traitements, notamment au moins un autre agent anti-diabétique, par exemple choisi parmi l'insuline, les sulfonyl-urée (par exemple glibenclamide, glimepiride, glipizide), les thiazoline-diones, les glinides, le DPP-IV inhibiteur (inhibiteur de dipeptidylpeptidase), le GLP-1 (Glucagon-like peptide-1) ou ses analogues.

De façon avantageuse, les composés de l'invention présentent une forte activité d'inhibition de la néoglucogenèse.
De façon avantageuse, les composés de l'invention présentent une activité de consommation du glucose.

L'invention concerne également l'utilisation d'un composé selon l'invention pour la préparation d'un médicament.
L'invention concerne également l'utilisation d'un composé selon l'invention pour la préparation d'un médicament pour le traitement de pathologies associées au syndrome d'insulino-résistance (ou syndrome X), notamment du diabète de type II.

La présente invention concerne également des compositions pharmaceutiques comprenant à titre de principe actif au moins un composé selon l'invention. Ces compositions peuvent également comprendre un véhicule et/ou excipient pharmaceutiquement acceptable.
Les compositions pharmaceutiques peuvent être sous toutes formes connues par l'homme du métier, notamment sous des formes destinées à l'administration par voie parentérale, orale, rectale, permuqueuse ou percutanée, de préférence par voie orale.
Les compositions selon l'invention seront présentées sous forme de solutés ou de suspensions injectables ou flacons multi-doses, sous forme de comprimés nus ou enrobés, de dragées, de capsules, de gélules, de pilules, de cachets, de poudres, de suppositoires ou de capsules rectales, de solutions de ou suspensions, pour l'usage percutané, dans un solvant polaire ou l'usage permuqueux.
Les excipients qui conviennent pour de telles administrations sont les dérivés de la cellulose ou la cellulose microcristalline, les carbonates alcalino-terreux, le phosphate de magnésium, les amidons, les amidons modifiés, le lactose pour des formes solides.
Pour l'usage rectal, le beurre de cacao ou les stéarates de polyéthylène glycol sont les excipients préférés.
Pour l'usage parentéral, l'eau, les solutés aqueux, le sérum physiologique, les solutés isotoniques sont les véhicules les plus commodément utilisés.
La posologie peut varier dans des limites importantes en fonction de l'indication thérapeutique, et de la voie d'administration, ainsi que de l'âge et du poids du sujet.

L'invention concerne également une composition comprenant à titre de principe actif au moins un composé selon l'invention en association avec au moins un autre agent anti-diabétique, par exemple choisi parmi l'insuline, les sulfonyl-urée (par exemple glibenclamide, glimepiride, glipizide), les thiazoline-diones, les glinides, le DPP-IV inhibiteur (inhibiteur de dipeptidylpeptidase), le GLP-1 (Glucagon-like peptide-1) ou ses analogues.

Ces compositions comprennent également un véhicule ou excipient pharmaceutiquement acceptable.

L'invention concerne également une méthode de traitement de pathologies associées au syndrome d'insulino-résistance (ou syndrome X), notamment du diabète de type II, comprenant l'administration, au patient qui en a besoin, d'une quantité suffisante d'au moins un composé selon l'invention avec un véhicule ou excipient pharmaceutiquement acceptable.

L'identification du patient qui a besoin du traitement ci-dessus indiqué est définie par l'homme du métier. Un vétérinaire ou un médecin peut identifier, par l'intermédiaire de tests cliniques, d'examen physique, de tests ou diagnostics biologiques et par l'historique familial et/ou médical, les sujets qui ont besoin d'un tel traitement.

On entend par quantité suffisante, une quantité de composé selon la présente invention efficace pour prévenir ou traiter des conditions pathologiques. La quantité suffisante peut être déterminée par l'homme du métier, par l'intermédiaire de technique conventionnelle et par l'observation des résultats obtenus dans des circonstances analogues. Pour déterminer la quantité suffisante différents facteurs doivent être pris en compte par l'homme du métier, notamment et sans y être limité : le sujet, sa taille, son âge, son état de santé général, la maladie impliquée et son degré de sévérité ; la réponse du sujet, le type de composé, le mode d'administration, la biodisponibilité de la composition administrée, le dosage, l'utilisation concomitante d'autres concomitante d'autres médicaments, etc. De préférence, 5 à 500 mg/jour du composé selon l'invention sont administrés au patient en une ou plusieurs prises, de préférence en une prise.
La présente invention concerne également une méthode de traitement de pathologies associées au syndrome d'insulino-résistance (ou syndrome X), notamment du diabète de type II, comprenant l'administration, au patient qui en a besoin, d'une quantité suffisante d'au moins un composé selon l'invention avec un véhicule ou excipient pharmaceutiquement acceptable et d'au moins un autre agent antidiabétique, par exemple choisi parmi l'insuline, les sulfonyl-urée (par exemple glibenclamide, glimepiride, glipizide), les thiazoline-diones, les glinides, le DPP-IV inhibiteur (inhibiteur de dipeptidylpeptidase), le GLP-1 (Glucagon-like peptide-1) ou ses analogues.

La présente invention va maintenant être décrite à l'aide d'exemples non limitatifs.

### Exemple 1. Schéma générale de préparation des composés pour lesquels R¹ représente C(O)CR³R⁴CR⁵R⁶C(O)OH

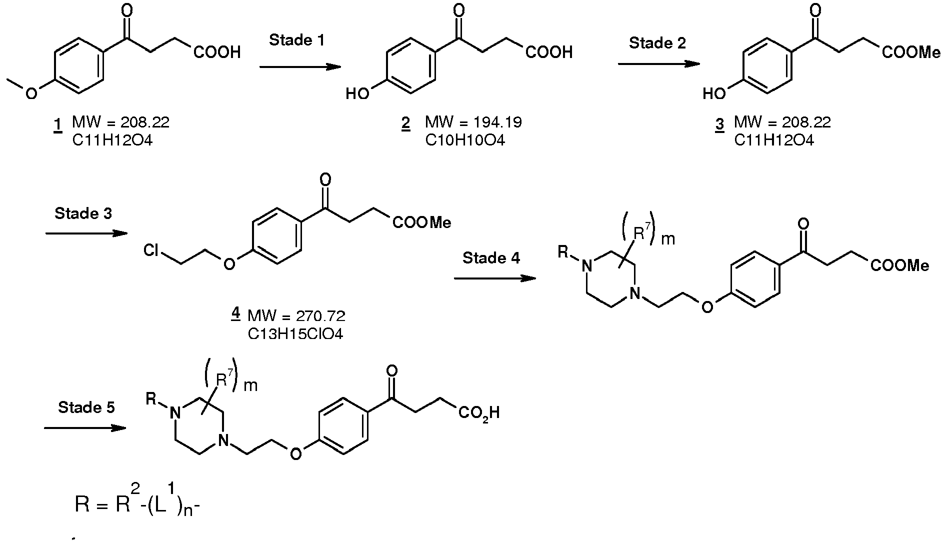

La première étape est une étape de déméthylation pouvant être réalisé par réaction de HBr en milieu acide acétique.
La seconde étape est une réaction d'estérification pouvant être réalisée par réaction avec le méthanol en présence d'acide sulfurique.
Les étapes 3 et 4 sont des étapes de substitution nucléophile.
L'étape 5 peut être réalisée notamment par hydrolyse acide ou saponification.

### Etape 1 : Déméthylation

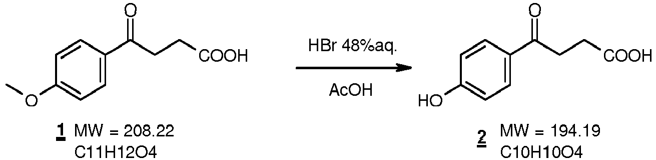

Equipement: Ballon de 1 L équipé d'une agitation magnétique et d'un réfrigérant - Bain d'huile.

Le 4-(4-methoxyphenyl)-4-oxobutanone **1** (40g) est mis en solution dans l'acide acétique (360ml) et l'acide bromhydrique à 48% aqueux (120ml). Le milieu réactionnel est chauffé à 130°C pour la nuit.
L'avancement de la réaction est contrôlé par CCM (éluant : Heptane/Acétate d'éthyle : 1/1). Après une nuit d'agitation dans ces conditions, on observe la disparition de l'éther de départ **1** au profit d'un produit plus polaire.
Le milieu réactionnel est concentré à sec sous pression réduite. Le résidu obtenu est repris par de l'eau et la phase aqueuse est extraite trois fois par de l'acétate d'éthyle. Une fois rassemblées, les phases organiques sont lavées une fois par une solution de chlorure de sodium saturée, séchées sur sulfate de magnésium, filtrées puis concentrées sous pression réduite pour fournir un solide (38g).
L'analyse RMN-1 H du brut réactionnel révèle la présence du phénol **2** attendu en mélange avec près de 6% d'éther de départ.
Rendement quantitatif.

### Etape 2 : Estérification

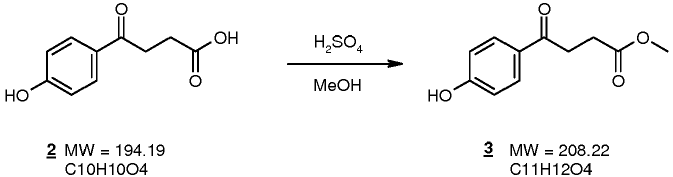

Equipement : Tricol de 1 L équipé d'une agitation magnétique, d'un réfrigérant et placé sous balayage d'azote - Bain d'huile.

Le dérivé 4-(4-hydroxyphényl)-4-oxobutanoique acide **2** (48g) est mis en solution dans le méthanol (480ml) avant de couler lentement l'acide sulfurique (48ml). La solution ainsi obtenue est chauffée à 70°C pour la nuit.
L'avancement de la réaction est contrôlé par CCM (éluant : Heptane/Acétate d'éthyle : 1/1). Après une nuit d'agitation dans ces conditions, on observe la disparition de l'acide de départ **2** au profit d'un produit moins polaire.
Le milieu réactionnel est concentré à sec sous pression réduite. Le résidu obtenu est repris par de l'eau (500ml) et par du dichlorométhane (500ml). Le milieu hétérogène est neutralisé, avec précaution, avec une solution saturée d'hydrogénocarbonate de sodium (pH 7-8). La phase aqueuse est ensuite extraite trois fois avec de l'acétate d'éthyle. Une fois rassemblées, les phases organiques sont lavées une fois par une solution de chlorure de sodium saturée, séchées sur sulfate de magnésium, filtrées puis concentrées sous pression réduite pour fournir un solide (49.6g).

L'analyse RMN-1 H du brut réactionnel révèle la présence de l'ester **3** attendu en mélange avec moins de 3% du composé 4-(4-méthoxyphényl)-4-oxobutanoate de méthyle. Rendement 96%.

### Etape 3 : Substitution nucléophile

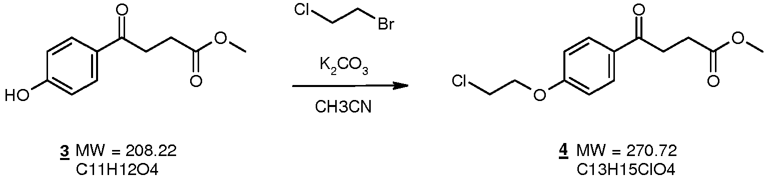

Equipement : Tricol de 1 L équipé d'une agitation magnétique, d'un réfrigérant et placé sous balayage d'azote - Bain d'huile.

Le 4-(4-hydroxyphényl)-4-oxobutanoate de méthyle **3** (49,6g) est mis en solution dans l'acétonitrile (400ml). Le carbonate de potassium (98,77g) est ajouté à la solution. Le milieu réactionnel est chauffé à 50°C avant de couler une solution de 1-bromo-2-chloroéthane (102,5g) dans l'acétonitrile (170ml). Le milieu réactionnel est chauffé à 80°C pour la nuit. L'avancement de la réaction est contrôlé par CCM (éluant : Heptane/Acétate d'éthyle : 7/3). Après 24H d'agitation dans ces conditions, on observe la disparition de composé **3** au profit d'un produit moins polaire.
Après retour à température ambiante, le milieu réactionnel est filtré pour éliminer le carbonate de potassium. Le carbonate de potassium est rincé avec de l'acétonitrile puis le filtrat est concentré à sec sous pression réduite. Le résidu obtenu est repris par de l'eau (500ml) et la phase aqueuse est extraite deux fois par de l'acétate d'éthyle (600ml). Les phases organiques sont rassemblées, lavées une fois avec une solution d'hydroxyde de sodium 1 M (400ml), séchées sur sulfate de magnésium, filtrées puis concentrées sous pression réduite pour fournir un solide beige (61,44g).
L'analyse RMN-1 H du brut réactionnel révèle la présence du dérivé chloré **4** attendu. Rendement 95%.

### Etape 4 : Substitution nucléophile

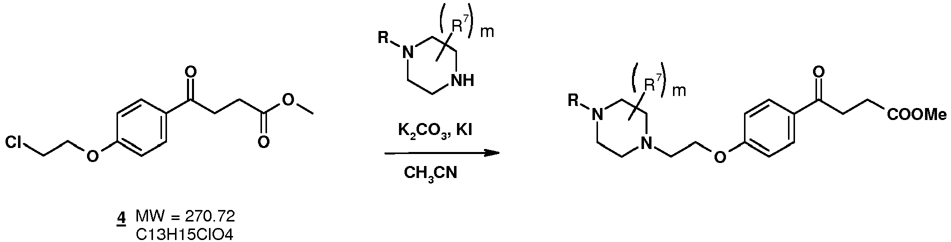

Equipement : Appareil Stem équipé d'un système de chauffage et d'une agitation orbitalaire, Réacteurs de 9ml - Manifold - système d'évaporation multivac - Appareil pour extraction Allexis

Sous balayage d'azote, le dérivé chloré **4** (500mg, 1,85mmol, 1 éq.) est réparti dans les différents réacteurs puis mis en solution dans l'acétonitrile (5ml) en présence de R-pipérazine (1,85mmol, 1 éq.), de carbonate de potassium (766mg, 5,54mmol, 3éq.) préalablement séché, de iodure de potassium (307mg, 1,85mmol, 1éq.). Après avoir réalisé un balayage d'azote, les réacteurs sont fermés et chauffés à 80°C.
Après 72 heures, le chauffage est arrêté. Après retour à température ambiante, les différents milieux réactionnels sont filtrés en parallèle sur cartouche Supelco reliée à un Manifold pour éliminer les sels inorganiques. Après rinçage par de l'acétonitrile, les filtrats sont concentrés à sec sous pression réduite à l'aide du multivac. Les résidus obtenus sont repris par de l'eau (20ml) et extraits trois fois, en parallèle sur appareil Allexis, par de l'acétate d'éthyle (10ml), Les différentes phases organiques rassemblées sont séchées sur sulfate de magnésium, filtrées puis concentrées sous pression réduite à l'aide du multivac.
Les différents bruts réactionnels sont purifiés par chromatographie sur colonne prépackée Redisep 40g, système Biotage SP4, en utilisant un gradient dichlorométhane / méthanol.

### Etape 5 : Hydrolyse acide

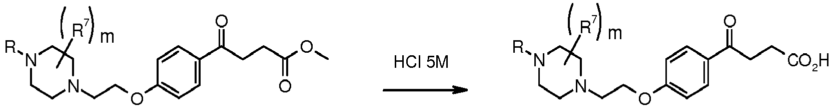

### Equipement : Syncore - Système de chauffage - agitation orbitalaire - Réacteurs de 50ml

Les différents esters sont mis en solution dans l'acide chlorhydrique 5 puis les solutions sont chauffées à 90°C pendant 2 heures. Un contrôle par CCM (Eluant : dichlorométhane/Méthanol 98/2, révélation UV) du milieu réactionnel de chaque réacteur permet de vérifier la disparition des esters au profit de produits plus polaires.
Les milieux réactionnels sont concentrés à sec sous pression réduite au multivac. Les solides obtenus sont triturés avec de l'acétone (10V) puis filtrés sur fritté et séchés sous vide.

### Etape 5 : Saponification

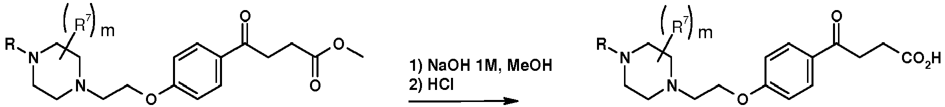

### Equipement : Radley équipé de réacteurs et muni d'une agitation magnétique.

Les différents esters sont mis en solution dans le méthanol (4V) en présence d'une solution de soude 1 M (8V). Après une nuit d'agitation dans ces conditions, un contrôle par CCM (Eluant : dichlorométhane/Méthanol 98/2, révélation UV) permet de vérifier la disparition des esters au profit de produits plus polaires.
Après concentration du méthanol, les différentes solutions aqueuses sont extraites par de l'acétate d'éthyle (5ml) puis acidifiées à pH 1 à l'aide d'une solution d'acide chlorhydrique 3M.
Les acides sont ensuite isolés par désalification en réalisant une élution des différentes solutions aqueuses sur résine Porapak Rxn.
Protocole de désalification :
- Conditionnement de la résine par 45ml de méthanol
- Déposer la phase aqueuse (4 à 5ml)
- Rincer par 100ml de méthanol
- Eluer par 100ml d'une solution d'ammoniaque 2M dans le méthanol
- Concentrer à sec sous pression réduite

Les solides obtenus après désalification sont triturés par de l'éther éthylique ou recristallisés dans l'acétonitrile.

### Exemple 2 : Obtention des composés selon l'invention

Les composés suivants ont été obtenus par mise en oeuvre du schéma général de l'exemple 1 (étape 5 hydrolyse acide)

| Rendement : 99% | Structure : | Composé 1 | |
|---|---|---|---|
| 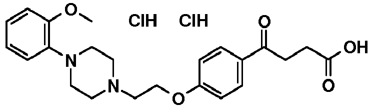 | | | |
| | | Masse moléculaire : | 485,41 |
| | | Formule brute : | C₂₃H₃₀Cl₂N₂O₅ |
| | | Forme/couleur : | Solide blanc |
| RMN-1 H (DMSO, 300MHz, δ ppm) : 2.56 (t, 2H); 3.10 (t, 2H); 3.21 (t, 2H); 3.33 (q, 2H); 3.50 (d, 2H); 3.61-3.66 (m, 4H); 3.80 (s, 3H); 4.58 (t, 2H); 6.90-7.11 (m, 4H); 7.14 (d, 2H); 8.00 (d, 2H); 11.21 (s large, 1 H) | | | |
| MS (ESI) : 413,12 (MH⁺); 411,10 (MH⁻) forme base | | | |
| | | | |

| Rendement : 85% | Structure : | Composé 2 | |
|---|---|---|---|
| 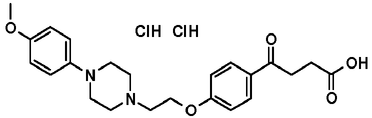 | | | |
| | | Masse moléculaire : | 485,41 |
| | | Formule brute : | C₂₃H₃₀Cl₂N₂O₅ |
| | | Forme/couleur : | Solide blanc |
| RMN-1 H (DMSO, 300MHz, δ ppm) : 2.56 (t, 2H); 3.12-3.23 (m, 4H); 3.23-3.35 (m, 2H); 3.59-3.70 (m, 9H); 4.58 (t, 2H); 6.87 (d, 2H); 7.00 (d, 2H); 7.14 (d, 2H); 7.99 (d, 2H); 11.38 (s large, 1 H) | | | |
| MS (ESI) : 413,12 (MH⁺); 411,10 (MH⁻) forme base | | | |
| | | | |

| Rendement : 95% | Structure : | Composé 3 | |
|---|---|---|---|
| 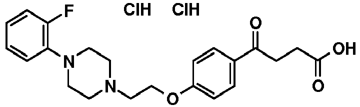 | | | |
| | | Masse moléculaire : | 473,38 |
| | | Formule brute : | C₂₂H₂₇Cl₂FN₂O₄ |
| | | Forme/couleur : | Solide blanc |
| RMN-1 H (DMSO, 300MHz, δ ppm) : 2.56 (t, 2H); 3.18-3.41 (m, 6H); 3.50 (d, 2H); 3.63-3.70 (m, 4H); 4.59 (t, 2H); 7.00-7.23 (m, 6H); 7.99 (d, 2H); 11.49 (s large, 1 H) | | | |
| MS (ESI) : 401,10 (MH⁺); 399,2 (MH⁻) forme base | | | |

| Rendement : 60% | Structure : | Composé 4 | |
|---|---|---|---|
| 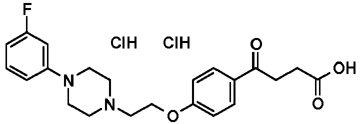 | | | |
| | | Masse moléculaire : | 473,38 |
| | | Formule brute : | C₂₂H₂₇Cl₂FN₂O₄ |
| | | Forme/couleur : | Solide blanc |
| RMN-1H (DMSO, 300MHz, δ ppm) : 2.56 (t, 2H); 3.18-3.41 (m, 6H); 3.59-3.66 (m, 4H); 3.70-3.93 (m, 2H); 4.53-4.59 (m, 2H); 6.63 (t, 1 H); 6.82 (d, 1 H); 6.87 (s, 1 H); 7.14 (d, 2H); 7.26 (q, 1 H); 8.00 (d, 2H); 11.25 (s large, 1 H); 12.15 (s large, 1 H) | | | |
| MS (ESI) : 401,20 (MH⁺); 399,3 (MH⁻) forme base | | | |

| Rendement : 96% | Structure : | Composé 5 | |
|---|---|---|---|
| 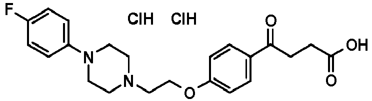 | | | |
| | | Masse moléculaire : | 473,38 |
| | | Formule brute : | C₂₂H₂₇Cl₂FN₂O₄ |
| | | Forme/couleur : | Solide blanc |
| RMN-1 H (DMSO, 300MHz, δ ppm) : 2.56 (t, 2H); 3.11-3.34 (m, 6H); 3.61-3.70 (m, 4H); 3.75 (d, 2H); 4.58 (t, 2H); 7.03-7.15 (m, 6H); 8.00 (d, 2H); 11.29 (s large, 1 H) | | | |
| MS (ESI) : 401,20 (MH⁺); 399,3 (MH⁻) forme base | | | |
| | | | |

| Rendement : 80% | Structure : | Composé 6 | |
|---|---|---|---|
| 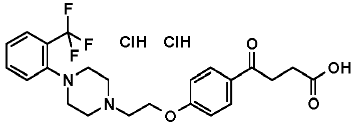 | | | |
| | | Masse moléculaire : | 523,38 |
| | | Formule brute : | C₂₃H₂₇Cl₂F₃N₂O₄ |
| | | Forme/couleur : | Solide blanc cassé |
| RMN-1 H (DMSO, 300MHz, δ ppm) : 2.56 (t, 2H); 3.10 (d, 2H); 3.21 (t, 2H); 3.28-3.38 (m, 4H); 3.62-3.72 (m, 4H); 4.54-4.60 (m, 2H); 7.14 (d, 2H); 7.41 (t, 1 H); 7.56 (d, 1 H); 7.72 (d, 2H); 8.00 (d, 2H); 11.32 (s large, 1 H); 12.15 (s large, 1 H) | | | |
| MS (ESI) : 451,20 (MH⁺); 449,20 (MH⁻) forme base | | | |
| | | | |

| Rendement : 30% | Structure : | Composé 7 | |
|---|---|---|---|
| 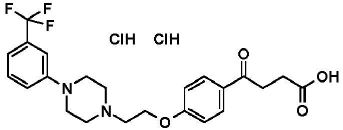 | | | |
| | | Masse moléculaire : | 523,38 |
| | | Formule brute : | C₂₃H₂₇Cl₂F₃N₂O₄ |
| | | Forme/couleur : | Solide blanc cassé |
| RMN-1 H (DMSO, 300MHz, δ ppm) : 2.56 (t, 2H); 3.18-3.28 (m, 6H); 3.62-3.72 (m, 4H); 3.93-4.11 (m, 2H); 4.53-4.58 (m, 2H); 7.13 (d, 2H); 7.15 (d, 1 H); 7.28 (d, 1 H); 7.29 (d, 1 H); 7.47 (t, 1 H); 8.00 (d, 2H); 11.21 (s large, 1 H); 12.15 (s large, 1 H) | | | |
| MS (ESI) : 451,20 (MH⁺); 449,20 (MH⁻) forme base | | | |

| Rendement : 20% | Structure : | Composé 8 | |
|---|---|---|---|
| 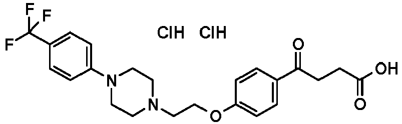 | | | |
| | | Masse moléculaire : | 523,38 |
| | | Formule brute : | C₂₃H₂₇Cl₂F₃N₂O₄ |
| | | Forme/couleur : | Solide blanc cassé |
| RMN-1 H (DMSO, 300MHz, δ ppm) : 2.57 (t, 2H); 3.21 (t, 2H); 3.29 (d, 4H); 3.62-3.72 (m, 4H); 4.02-4.10 (m, 2H); 4.52-4.58 (m, 2H); 7.11-7.15 (m, 4H); 7.58 (d, 2H); 8.00 (d, 2H); 11.10 (s large, 1 H); 12.18 (s large, 1 H) | | | |
| MS (ESI) : 451,20 (MH⁺); 449,20 (MH⁻) forme base | | | |
| | | | |

| Rendement : 78% | Structure : | Composé 9 | |
|---|---|---|---|
| 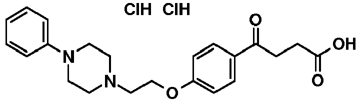 | | | |
| | | Masse moléculaire : | 455,39 |
| | | Formule brute : | C₂₂H₂₈Cl₂N₂O₄ |
| | | Forme/couleur : | Solide blanc |
| RMN-1 H (DMSO, 300MHz, δ ppm) : 2.56 (t, 2H); 3.13-3.35 (m, 6H); 3.60-3.68 (m, 4H); 3.83 (d, 2H); 4.58 (t, 2H); 6.86 (t, 1 H); 7.00 (d, 2H); 7.14 (d, 2H); 7.26 (t, 2H); 8.00 (d, 2H); 11.36 (s large, 1 H) | | | |
| MS (ESI) : 383,20 | (MH⁺); 381,20 (MH⁻) forme | base | |
| | | | |

| Rendement : 93% | Structure : | Composé 10 | |
|---|---|---|---|
| 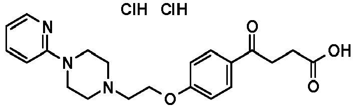 | | | |
| | | Masse moléculaire : | 456,37 |
| | | Formule brute : | C₂₁H₂₇Cl₂N₃O₄ |
| | | Forme/couleur : | Solide blanc |
| RMN-1 H (DMSO, 300MHz, δ ppm) : 2.56 (t, 2H); 3.18-3.32 (m, 4H); 3.42-3.52 (m, 2H); 3.62-3.72 (m, 4H); 4.45 (d, 2H); 4.55 (t, 2H); 6.88 (t, 1H); 7.12-7.18 (m, 3H); 7.80-7.85 (m, 1H); 8.00 (d, 2H); 8.14 (d, 1 H); 11.24 (s large, 1 H) | | | |
| MS (ESI) : 384,30 (MH⁺); 382,20 (MH⁻) forme base | | | |
| | | | |

| Rendement : 99% | Structure : | Composé 11 | |
|---|---|---|---|
| 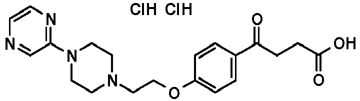 | | | |
| | | Formule brute : | C₂₀H₂₆Cl₂N₄O₄ |
| | | Forme/couleur : | Solide jaune |
| RMN-1 H (DMSO, 300MHz, δ ppm) : 2.56 (t, 2H); 3.18-3.28 (m, 4H); 3.45 (t, 2H); 3.58-3.67 (m, 4H); 4.47 (d, 2H); 4.57 (t, 2H); 7.13 (d, 2H); 7.96 (d, 1 H); 8.00 (d, 2H); 8.18 (dd, 1 H); 8.45 (dd, 1 H); 11.52 (s large, 1 H) | | | |
| MS (ESI) : 385,20 (MH⁺); 383,20 (MH⁻) forme base | | | |

| Rendement : 99% | Structure : | Composé 12 | |
|---|---|---|---|
| 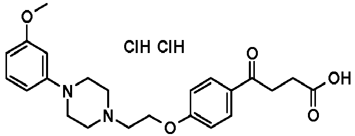 | | | |
| | | Masse moléculaire : | 485,41 |
| | | Formule brute : | C₂₃H₃₀Cl₂N₂O₅ |
| | | Forme/couleur : | Solide blanc |
| RMN-1 H (DMSO, 300MHz, δ ppm) : 2.56 (t, 2H); 3.12-3.33 (m, 6H); 3.61-3.67 (m, 4H); 3.73 (s, 3H); 3.84 (d, 2H); 4.57 (t, 2H); 6.45 (dd, 1 H); 6.53 (s, 1 H); 6.58 (dd, 1 H); 7.10-7.19 (m, 3H); 8.00 (d, 2H); 11.21 (s large, 1 H) | | | |
| MS (ESI) : 413,20 (MH⁺); 411,20 (MH⁻) forme base | | | |
| | | | |

| Rendement : 70% | Structure : | Composé 13 | |
|---|---|---|---|
| 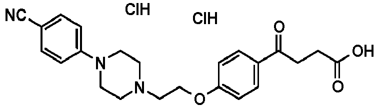 | | | |
| | | Masse moléculaire : | 480,40 |
| | | Formule brute : | C₂₃H₂₇Cl₂N₃O₄ |
| | | Forme/couleur : | Solide blanc |
| RMN-1 H (DMSO, 300MHz, δ ppm) : 2.57 (t, 2H); 3.20 (t, 2H); 3.25-3.35 (m, 4H); 3.62-3.66 (m, 4H); 4.05-4.12 (m, 2H); 4.52-4.54 (m, 2H); 7.12 (dd, 4H); 7.67 (d, 2H); 8.00 (d, 2H); 10.79 (s large, 1 H); 12.10 (s large, 1 H) | | | |
| MS (ESI) : 408,10 (MH⁺); 406,00 (MH⁻) forme base | | | |
| | | | |

| Rendement : 50% | Structure : | Composé 14 | |
|---|---|---|---|
| 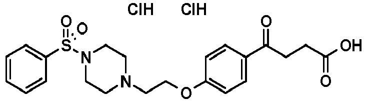 | | | |
| | | Masse moléculaire : | 519,45 |
| | | Formule brute : | C₂₂H₂₈Cl₂N₂O₆S |
| | | Forme/couleur : | Solide blanc |
| RMN-1 H (DMSO, 300MHz, δ ppm) : 2.56 (t, 2H); 2.76 (t, 2H); 3.19 (t, 2H); 3.25-3.31 (m, 2H); 3.53-3.84 (m, 6H); 4.46 (t, 2H); 7.07 (d, 2H); 7.67-7.80 (m, 5H); 7.97 (d, 2H); 11.08 (s large, 1H); 12.13 (s large, 1H) | | | |
| MS (ESI) : 447,00 (MH⁺); 444,90 (MH⁻) forme base | | | |
| | | | |

| Rendement : 67% | Structure : | Composé 15 | |
|---|---|---|---|
| 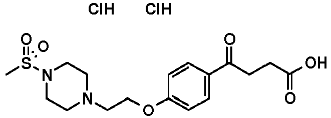 | | | |
| | | Masse moléculaire : | 457,38 |
| | | Formule brute : | C₁₇H₂₆Cl₂N₂O₆S |
| | | Forme/couleur : | Solide blanc |
| RMN-1 H (DMSO, 300MHz, δ ppm) : 2.56 (t, 2H); 3.02 (s, 3H); 3.20 (t, 2H); 3.26-3.76 (m, 10H); 4.53 (t, 2H); 7.11 (d, 2H); 7.98 (d, 2H); 11.38 (s large, 1H); 12.15 (s large, 1H) | | | |
| MS (ESI) : 385,10 (MH⁺); 383,10 (MH⁻) forme base | | | |

| Rendement : 74% | Structure : | Composé 16 | |
|---|---|---|---|
| 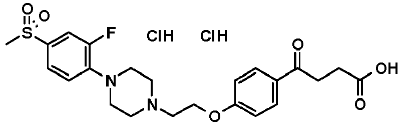 | | | |
| | | Masse moléculaire : | 551,47 |
| | | Formule brute : | C₂₃H₂₉Cl₂FN₂O₆S |
| | | Forme/couleur : | Solide blanc |
| RMN-1 H (DMSO, 300MHz, δ ppm) : 2.56 (t, 2H); 3.19-3.23 (m, 6H); 3.31-3.37 (m, 4H); 3.64-3.75 (m, 5H); 4.53 (t, 2H); 7.14 (d, 2H); 7.33 (t, 1 H); 7.68 (d, 1 H); 7.74 (d, 1 H); 8.00 (d, 2H); 10.80 (s large, 1 H); 12.14 (s large, 1 H) | | | |
| MS (ESI) : 479,00 (MH⁺); 476,90 (MH⁻) forme base | | | |
| | | | |

| Rendement : 60% | Structure : | Composé 17 | |
|---|---|---|---|
| 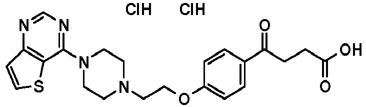 | | | |
| | | Masse moléculaire : | 513,45 |
| | | Formule brute : | C₂₂H₂₆Cl₂N₄O₄S |
| | | Forme/couleur : | Solide blanc |
| RMN-1 H (DMSO, 300MHz, δ ppm) : 2.57 (t, 2H); 3.21 (t, 2H); 3.37-4.05 (m, 8H); 4.57 (t, 2H); 4.87-4.90 (m, 2H); 7.14 (d, 2H); 7.65 (d, 1 H); 8.00 (d, 2H); 8.59 (d, 1 H); 8.89 (s, 1 H); 12.10 (s large, 1 H) | | | |
| MS (ESI) : 441,00 (MH⁺); 439,00 (MH⁻) forme base | | | |
| | | | |

| Rendement : 69% | Structure : | Composé 18 | |
|---|---|---|---|
| 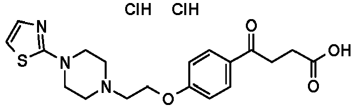 | | | |
| | | Masse moléculaire : | 462,40 |
| | | Formule brute : | C₁₉H₂₅Cl₂N₃O₄S |
| | | Forme/couleur : | Solide blanc |
| RMN-1 H (DMSO, 300MHz, δ ppm) : 2.57 (t, 2H); 3.21 (t, 2H); 3.29-3.38 (m, 2H); 3.54 (t, 2H); 3.63-3.70 (m, 4H); 4.05 (d, 2H); 4.54 (t, 2H); 7.01 (d, 1 H); 7.13 (d, 2H); 7.26 (d, 1 H); 8.00 (d, 2H); 11.21 (s large, 1 H) | | | |
| MS (ESI) : 390,00 (MH⁺); 388,00 (MH⁻) forme base | | | |

### Les composés suivants ont été obtenus par mise en oeuvre du schéma général de l'exemple 1 (étape 5 saponification)

| Rendement : 55% | Structure : | Composé 19 | |
|---|---|---|---|
| 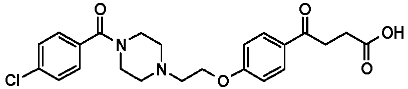 | | | |
| | | Masse moléculaire : | 444,92 |
| | | Formule brute : | C₂₃H₂₅ClN₂O₅ |
| | | Forme/couleur : | Solide blanc |
| RMN-1 H (DMSO, 300MHz, δ ppm) : 2.41 (t, 2H); 2.44-2.57 (m, 4H); 2.76 (t, 2H); 3.11 (t, 2H); 3.28-3.38 (m, 2H); 3.59-3.66 (m, 2H); 4.17 (t, 2H); 7.04 (d, 2H); 7.47 (dd, 4H); 7.93 (d, 2H) | | | |
| MS (ESI) : 444,90 (MH⁺); 442,90 (MH⁻) | | | |
| | | | |

| Rendement : 50% | Structure : | Composé 20 | |
|---|---|---|---|
| 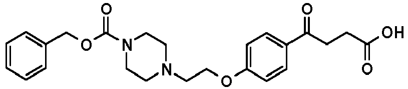 | | | |
| | | Masse moléculaire : | 440,50 |
| | | Formule brute : | C₂₄H₂₈N₂O₆ |
| | | Forme/couleur : | Solide blanc |
| RMN-1 H (DMSO, 300MHz, δ ppm) : 2.40 (t, 2H); 2.44-2.51 (m, 4H); 2.74 (t, 2H); 3.09 (t, 2H); 3.36-3.44 (m, 4H); 4.16 (t, 2H); 5.07 (s, 2H); 7.04 (d, 2H); 7.29-7.40 (m, 5H); 7.92 (d, 2H) | | | |
| MS (ESI) : 440,90 | (MH⁺); 438,90 (MH⁻) | | |
| | | | |

| Rendement : 47% | Structure : | Composé 21 | |
|---|---|---|---|
| 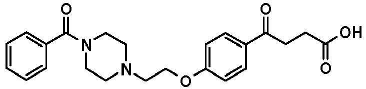 | | | |
| | | Masse moléculaire : | 410,47 |
| | | Formule brute : | C₂₃H₂₆N₂O₅ |
| | | Forme/couleur : | Solide blanc |
| RMN-1 H (DMSO, 300MHz, δ ppm) : 2.37 (t, 2H); 2.44-2.58 (m, 4H); 2.76 (t, 2H); 3.09 (t, 2H); 3.29-3.39 (m, 2H); 3.57-3.66 (m, 2H); 4.18 (t, 2H); 7.04 (d, 2H); 7.36-7.47 (m, 5H); 7.92 (d, 2H) | | | |
| MS (ESI) : 470,90 (MH⁺); 408,90 (MH⁻) | | | |
| | | | |

| Rendement : 21% | Structure : | Composé 22 | |
|---|---|---|---|
| 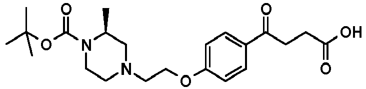 | | | |
| | | Masse moléculaire : | 420,51 |
| | | Formule brute : | C₂₂H₃₂N₂O₆ |
| | | Forme/couleur : | Solide blanc |
| RMN-1 H (DMSO, 300MHz, δ ppm) : 1.12 (d, 3H); 1.39 (s, 9H); 1.97 (td, 1 H); 2.15 (dd, 1 H); 2.39 (t, 2H); 2.65-2.78 (m, 3H); 2.84-3.02 (m, 2H); 3.10 (t, 2H); 3.66 (d, 1H); 4.03-4.11 (m, 1 H); 4.17 (t, 2H); 7.05 (d, 2H); 7.93 (d, 2H) | | | |
| MS (ESI) : 421,00 (MH⁺); 419,00 (MH⁻) | | | |

| Rendement : 20% | Structure : | Composé 23 | |
|---|---|---|---|
| 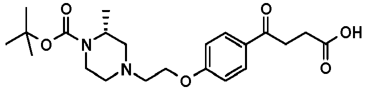 | | | |
| | | Masse moléculaire : | 420,51 |
| | | Formule brute : | C₂₂H₃₂N₂O₆ |
| | | Forme/couleur : | Solide blanc |
| RMN-1 H (DMSO, 300MHz, δ ppm) : 1.12 (d, 3H) ; 1.39 (s, 9H); 1.97 (td, 1 H); 2.15 (dd, 1 H); 2.38 (t, 2H); 2.66-2.77 (m, 3H); 2.84-3.02 (m, 2H); 3.09 (t, 2H); 3.65 (d, 1 H); 4.02-4.10 (m, 1 H); 4.16 (t, 2H); 7.03 (d, 2H); 7.93 (d, 2H) | | | |
| MS (ESI) : 421,00 (MH⁺); 419,00 (MH⁻) | | | |

### Exemple 3 : Schéma général de préparation de composés pour lesquels R¹ représente -

C(OH)HCR³R⁴CR⁵R⁶C(O)OH ou 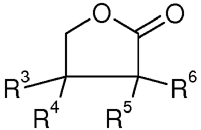 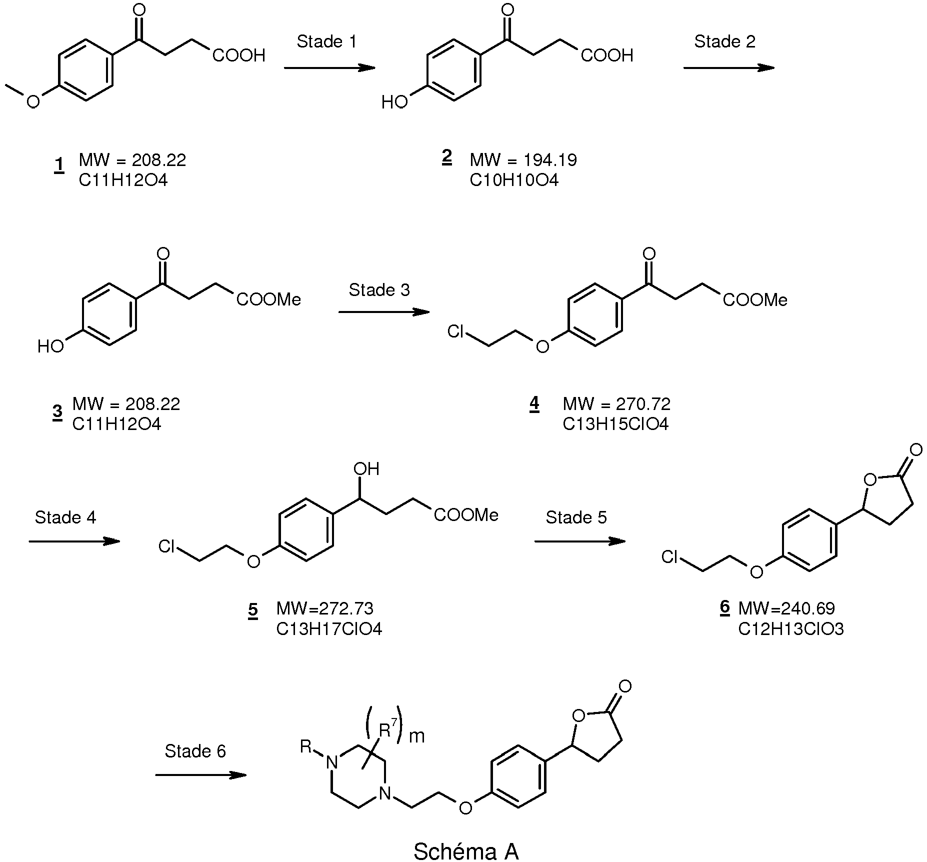 Ou

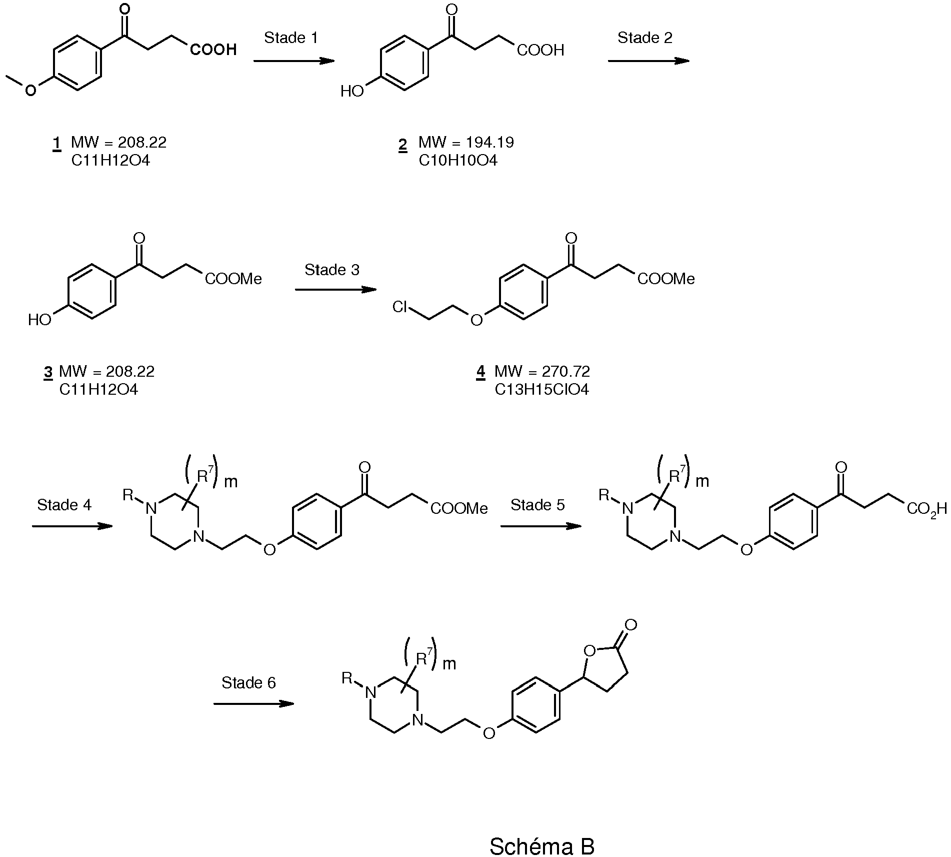

Les étapes 1 à 3 des schémas A et B correspondent aux étapes 1 à 3 du schéma de l'exemple 1. Les étapes 4 et 5 du schéma B correspondent aux étapes 4 et 5 du schéma de l'exemple 1.

### Etape 4 du schéma A : Réduction de la fonction 4-Oxo

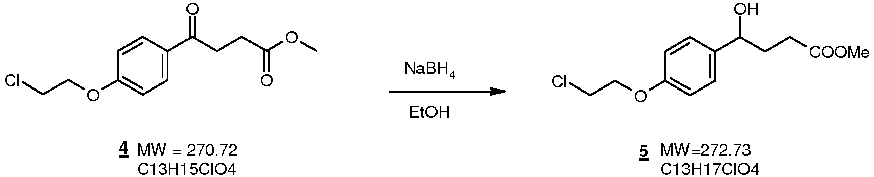

### Equipement: Tricol de 100ml équipé d'une agitation magnétique et placé sous balayage d'azote.

Le dérivé chloré **4** (5g, 18,5mmol, 1 éq.) est mis en suspension dans l'éthanol absolu (50ml). Le tétraborohydrure de sodium (2,8g, 74mmmol), 4éq.) est ajouté, par portions sur une période de 15 minutes, à la suspension préalablement refroidie à 0°C à l'aide d'un bain de saumure. A la fin de l'addition le bain de saumure est retiré et le milieu réactionnel est laissé sous agitation à température ambiante.
L'avancement de la réaction est contrôlé par CCM (éluant : Heptane/Acétate d'éthyle : 1/1). Après une heure et quart d'agitation on observe la disparition de la cétone de départ **4** au profit de la formation de 2 produits plus polaires.
L'éthanol est éliminé par évaporation sous pression réduite. Le résidu obtenu est repris par un mélange eau/glace et acidifié, à pH2, par addition lente d'une solution d'acide chlorhydrique 3N. La phase aqueuse est ensuite extraite trois fois par de l'acétate d'éthyle. Une fois rassemblées, les phases organiques sont lavées une fois par une solution de chlorure de sodium saturée, séchées sur sulfate de magnésium, filtrées puis concentrées sous pression réduite pour fournir une huile jaune (4,5g).
L'analyse RMN-1 H du brut réactionnel révèle la présence de l'alcool **5** attendu en mélange avec la forme lactone **6**. Le brut réactionnel est engagé en cyclisation sans étape de purification.

### Etape 5 du schéma A : Lactonisation

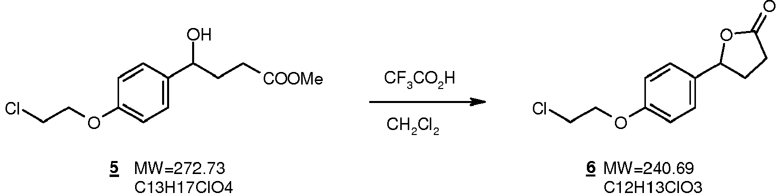

### Equipement : Tricol de 100ml équipé d'une agitation magnétique, d'un réfrigérant et placé sous balayage d'azote.

Le mélange alcool/lactone **5**/**6** (4,5g) est mis en solution dans le dichlorométhane (30ml) en présence d'acide trifluoroacétique (0,2ml). La solution est chauffée au reflux pendant 6 heures.
L'avancement de la réaction est contrôlé par CCM (éluant : Heptane/Acétate d'éthyle : 1/1). La réaction n'est pas totale mais n'évolue plus.
Le chauffage est coupé. Après retour du milieu réactionnel à température ambiante, la phase dichlorométhane est lavée successivement par une solution d'hydrogénocarbonate de sodium saturée puis à l'eau. La phase organique est séchée sur sulfate de magnésium, filtrée puis concentrée sous pression réduite pour fournir une cire blanche (3,8g).
Le résidu cireux est purifié par chromatographie sur colonne prépackée (80g). Les composés à séparer sont élués en utilisant un gradient heptane / acétate d'éthyle.

Le composé souhaité est isolé sous la forme d'un solide blanc (1,3g).
Rendement sur les deux étapes 29%

### Etape 6 du schéma A : Réaction de substitution nucléophile

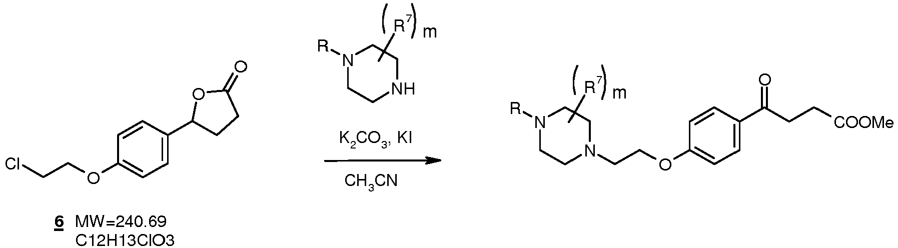

### Equipement : Tube de schlenk (25ml) équipé d'une agitation magnétique.

Sous balayage d'azote, le dérivé chloré **6** (1 éq.) est mis en solution dans l'acétonitrile (4ml) en présence de R-pipérazine (10V), de carbonate de potassium (3éq.) préalablement séché, de iodure de potassium (1éq.). Après avoir réalisé un balayage d'azote, le réacteur est fermé et chauffé à 80°C pendant 72 heures.
Après 72 heures, le chauffage est arrêté. Après retour à température ambiante, le milieu réactionnel est filtré sur cartouche Supelco pour éliminer les sels inorganiques. Après rinçage par de l'acétonitrile, le filtrat est concentré à sec sous pression réduite. Le résidu obtenu est repris par de l'acétate d'éthyle et lavé deux fois par de l'eau. La phase organique est séchée sur sulfate de magnésium, filtrée puis concentrée sous pression réduite.
Le brut réactionnel est purifié par chromatographie sur colonne prépackée Redisep 40g, système Biotage SP4, en utilisant un gradient dichlorométhane / méthanol.

### Exemple 4 : Obtention de composés de formule (I) selon l'invention

Les composés qui suivent ont été obtenus par mise en oeuvre du procédé selon le schéma A de l'exemple 3.

| Rendement : 70% | Structure : | Composé 24 | |
|---|---|---|---|
| 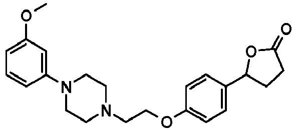 | | | |
| | | Masse moléculaire : | 396,49 |
| | | Formule brute : | C₂₃H₂₈N₂O₄ |
| | | Forme/couleur : | Huile jaune |
| RMN-1 H (DMSO, 300MHz, δ ppm) : 2.15-2.28 (m, 1H); 2.58-2.71 (m, 3H); 2.75-2.85 (m, 4H); 2.91 (t, 2H); 3.26 (dd, 4H); 3.80 (s, 3H); 4.19 (t, 2H); 5.45-5.51 (m, 1 H); 6.44 (dd, 1 H); 6.48 (t, 1 H); 6.57 (dd, 1 H); 6.95 (dd, 2H); 7.19 (t, 1 H); 7.28 (d, 2H) | | | |
| MS (ESI+) : 397,3 (MH⁺) | | | |

| Rendement : 30% | Structure : | Composé 25 | |
|---|---|---|---|
| 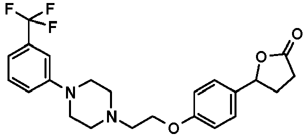 | | | |
| | | Masse moléculaire : | 434,46 |
| | | Formule brute : | C₂₃H₂₅F₃N₂O₃ |
| | | Forme/couleur : | Solide vert pâle |
| RMN-1 H (DMSO, 300MHz, δ ppm) : 2.16-2.29 (m, 1H); 2.58-2.71 (m, 3H); 2.78-2..83 (m, 4H); 2.93 (t, 2H); 3.31 (dd, 4H); 4.20 (t, 2H); 5.46-5.51 (m, 1H); 6.96 (d, 2H); 7.06-7.14 (m, 3H); 7.29 (d, 2H); 7.37 (t, 1 H) | | | |
| MS (ESI+) : 435,3 (MH⁺) | | | |

Le composé qui suit a été obtenu par mise en oeuvre du procédé selon le schéma B de l'exemple 3, l'étape 6 étant réalisé comme suit :

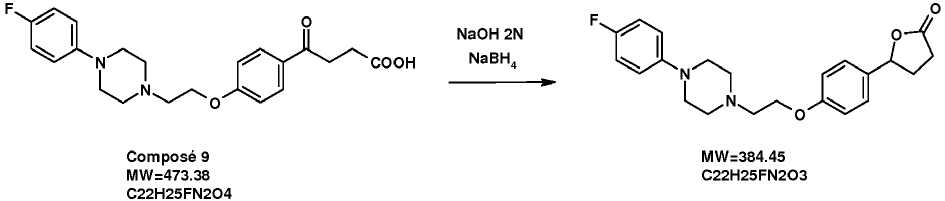

### Equipement : Tricol de 25ml équipé d'une agitation magnétique et placé sous balayage d'azote.

Le composé 9 (0,25g, 0,528mmol, 1 éq.) est mis en suspension dans l'eau (7,5ml) puis en solution par l'ajout d'une solution de soude 2N (1,5ml). Le tétraborohydrure de sodium (24mg, 0,324mmol, 1,2éq.) est ajouté à la solution. Le milieu réactionnel est laissé sous agitation à température ambiante pour la nuit.
L'avancement de la réaction est contrôlé par CCM (éluant : dichlorométhane/méthanol : 95/5).
Le milieu réactionnel est lavé par du dichlorométhane. La phase aqueuse est acidifiée à pH1 à l'aide d'une solution molaire de chlorure d'acide puis extraite deux fois par du dichlorométhane. Le produit étant partiellement soluble dans l'eau, la phase aqueuse acide est concentrée à sec sous pression réduite. Les sels sont repris dans un minimum d'eau et le produit est extrait deux fois par du dichlorométhane. Les différentes phases de dichlorométhane sont rassemblées, séchées sur sulfate de magnésium, filtrées et concentrées sous pression réduite pour fournir une meringue blanche (0,2g).
Le brut réactionnel est purifié par chromatographie sur colonne prépackée Redisep 15g, système Biotage SP4, en utilisant un gradient dichlorométhane / méthanol.
Rendement 60%

| Rendement : 60% | Structure : | Composé 26 | |
|---|---|---|---|
| 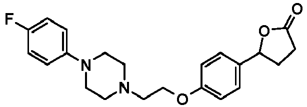 | | | |
| | | Masse moléculaire : | 384,45 |
| | | Formule brute : | C₂₂H₂₅FN₂O₃ |
| | | Forme/couleur : | Solide blanc |
| RMN-1 H (DMSO, 300MHz, δ ppm) : 2.14-2.25 (m, 1H); 2.58-2.70 (m, 3H); 3.30-3.50 (m, 10H); 4.48-4.55 (m, 2H); 5.44-5.50 (m, 1 H); 6.89-7.03 (m, 6H); 7.29 (d, 1 H); 7.31 (d, 1 H) | | | |
| MS (ESI+) : 385,3 (MH⁺) | | | |

### Exemple 5 : Etude de l'activité antidiabétiaue chez la souris db/db

L'activité antidiabétique des composés de formule (I) par voie orale sur un modèle animal de diabète de type 2, la souris db/db a été testé.
Les souris diabétiques sont utilisées à l'âge de 8 semaines; elles présentent alors une hyperglycémie très élevée. La stabulation des animaux est réalisée au minimum pendant une semaine après réception et jusqu'au jour de l'expérimentation dans une animalerie à température régulée à 21-22 °C et soumise à un cycle fixe de lumière (de 7 h à 19 h) et d'obscurité (de 19 h à 7 h). Leur alimentation a consisté en un régime d'entretien ; eau et nourriture ont été fournies « ad libitum ».
Les souris sont traitées par voie orale avec le produit à tester (200 mg/kg per os) en suspension dans un mélange d'eau et de méthyl cellulose ou le véhicule (mélange d'eau et de méthyl cellulose) le matin du jour de l'expérimentation. Juste avant l'administration du produit et une et deux heures après l'administration du produit, un prélèvement sanguin (une goutte de sang) est fait suite à une petite incision de la queue pour une détermination de la glycémie.
Cette glycémie est mesurée à l'aide d'un glucomètre (Lifescan OneTouch Ultra, LifeScan, Johnson-Johnson Company) et de bandelettes de mesure de glycémie OneTouch Ultra.
Le tableau 1 rassemble les résultats obtenus. Ces résultats expriment les pourcentages de chute de la glycémie initiale. Pour comparaison, la chute de la glycémie est également rapportée pour des animaux témoins n'ayant reçu que le véhicule et pour la metformine.

| | Chute de la glycémie (en % de la valeur initiale) | |
|---|---|---|
| | Au bout d'1 heure | Au bout de 2 heures |
| Témoins | 4 | 12 |
| Composé 2 | 7 | 12 |
| Composé 3 | 23 | 29 |
| Composé 4 | 9 | 20 |
| Composé 5 | 5 | 23 |
| Composé 9 | 7 | 34 |
| Composé 10 | 23 | 39 |
| Composé 12 | 19 | 32 |
| Metformine | 22 | 27 |

Ces résultats montrent l'efficacité des composés de formule (I) pour induire une diminution de la glycémie chez les animaux diabétiques.

## Revendications

1. Composé de formule (I) 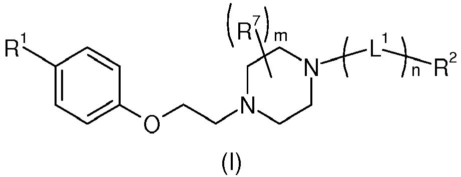 dans laquelle :
▪ R¹ représente :
- un groupe -C(O)CR³R⁴CR⁵R⁶C(O)OH ; ou
- un groupe 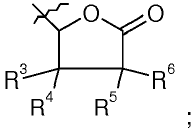
▪ n représente 0 ou 1 ;
▪ m représente 0 ou 1 ;
▪ L¹ représente un groupe -C(O)- ; -C(O)O- ou -S(O)₂- ;
▪ R² représente :
- un groupe phényle non substitué ou substitué par un ou plusieurs substituants, identiques ou différents choisis parmi :
- un groupe alkoxy en C₁ à C₆, linéaire ou ramifié ;
- un atome d'halogène ;
- un groupe hydrocarboné, linéaire ou ramifié, en C₁ à C₅, substitué par un ou plusieurs atomes d'halogène;
- un groupe hydrocarboné, linéaire ou ramifié, en C₁ à C₅ ;
- un groupe cyano (-CN) ;
- un groupe alkyle sulfonyle (-S(O)₂-alkyle), dans lequel l'alkyle est linéaire ou ramifié, en C₁ à C₅ ;
- un groupe hétérocycle aromatique en 5, 6 ou 7 membres, comprenant 1 ou 2 hétéroatomes identiques ou différents choisis parmi azote, soufre et oxygène;
- un groupe polyhétérocycle aromatique en 9 ou 10 membres comprenant 1, 2 ou 3 hétéroatomes, identiques ou différents choisis parmi azote, soufre et oxygène ;
- un groupe- L² -phényle ;
ou
- un groupe hydrocarboné, linéaire ou ramifié, en C₁ à C₅ ;
▪ L² représente un alkyle, linéaire ou ramifié en C₁ à C₅ ;
▪ R³, R⁴, R⁵ et R⁶ représentant un atome d'hydrogène ;
▪ R⁷ représente un alkyle, linéaire ou ramifié, en C₁ à C₅ ;
leurs énantiomères, diastéréoisomères et leurs sels d'addition avec des bases ou des acides pharmaceutiquement acceptables.

2. Composé selon la revendication 1 pour lequel R² représente :
- un phényl, non substitué ou substitué par un ou plusieurs substituants, identiques ou différents, choisi parmi :
• un groupe alkoxy en C₁ à C₆, linéaire ou ramifié ;
• un atome d'halogène ;
• un groupe alkyle en C₁ à C₅, linéaire ou ramifié ;
• un groupe alkyle en C₁ à C₅, linéaire ou ramifié, substitué par un ou plusieurs atomes d'halogène ;
• un groupe cyano (-CN) ;
• un groupe alkylsulfonyle (-S(O)₂-alkyl), dans lequel l'alkyle est linéaire ou ramifié, en C₁ à C₅ ; ou
- un hétéroaryl monocyclique ou polycyclique choisi parmi 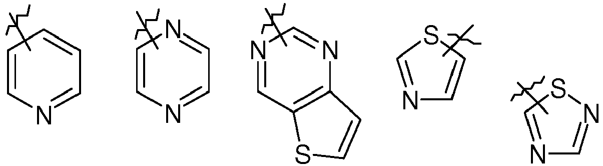
- un groupe -Alk-phényle Alk étant un alkyle, linéaire ou ramifié en C₁ à C₅ ; ou
- un groupe alkyle, linéaire ou ramifié, en C₁ à C₅.

3. Composé selon l'une des revendications 1 ou 2, dans lequel R¹ représente un groupe - C(O)CR³R⁴CR⁵R⁶C(O)OH ;

4. Composé selon l'une des revendications 1 à 3, dans lequel n=0

5. Composé selon l'une des revendications 1 à 3, dans lequel n=1.

6. Composé selon l'une des revendications 1 à 5, dans lequel m=0.

7. Composé selon l'une quelconque des revendications 1 à 4, dans lequel :
R¹ représente un groupe -C(O)CR³R⁴CR⁵R⁶C(O)OH ;
R² représente un groupe choisi parmi :
- un groupe phényle, non substitué ou substitué par un ou plusieurs substituants choisis parmi un groupe alkoxy en C₁ à C₆, linéaire ou ramifié ; un atome d'halogène ;
- un groupe hétérocycle aromatique en 6 membres comprenant 1 ou 2 hétéroatomes, identiques ou différents, choisis parmi azote, soufre et oxygène.

8. Procédé de préparation (P1) d'un composé selon l'une quelconque des revendications 1 à 7 comprenant :
(a) la protection de la fonction acide d'un composé de formule (II) 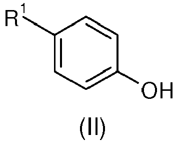 dans laquelle R¹ représente -C(O)CR³R⁴CR⁵R⁶C(O)OH ; R³, R⁴, R⁵ et R⁶ sont tels que définis par les revendications 1 à 7
(b) la réaction du composé obtenu à l'étape (a) avec un composé de formule R⁹-(CH₂)₂-R⁸ dans laquelle R⁸ et R⁹, identiques ou différents représentent un groupe partant, de préférence R⁹ est meilleur nucléofuge que R⁸ ;
(c) la réaction du composé obtenu à l'étape (b) avec un composé de formule (III) 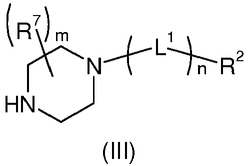 dans laquelle L¹, n, m, R² et R⁷ sont tels que définis par les revendications 1 à 7 ;
(d) déprotection du composé obtenu à l'étape (c).

9. Procédé de préparation (P2) d'un composé selon l'une quelconque des revendications 1 à 7 comprenant :
(i) la réaction d'un composé de formule (III) 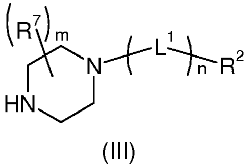 dans laquelle L¹, n, m, R² et R⁷ sont tels que définis par les revendications 1 à 7 avec un composé de formule R⁹-(CH₂)₂-R⁸, dans laquelle R⁸ et R⁹ sont tels que définis à la revendication 8 ;
(ii) la protection de la fonction acide d'un composé de formule (II) 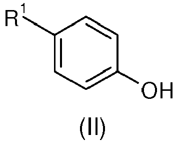 5 dans laquelle R¹ représente -C(O)CR³R⁴CR⁵R⁶C(O)OH ; R³, R⁴, R⁵ et R⁶ sont tels que définis aux revendications 1 à 7
(iii) la réaction entre le composé obtenu à l'étape (i) et le composé obtenu à l'étape (ii) ;
(iv) la déprotection du composé obtenu à l'étape (iii).

10. Composé selon l'une quelconque des revendications 1 à 7 en tant que médicament.

11. Composé selon l'une quelconque des revendications 1 à 7 pour son utilisation pour le traitement de pathologies associées au syndrome d'insulinorésistance.

12. Composé pour son utilisation selon la revendication 11 pour le traitement du diabète de type (II).

13. Composition pharmaceutique comprenant à titre de principe actif au moins un composé selon l'une quelconque des revendications 1 à 7.

14. Composition pharmaceutique selon la revendication 13, comprenant en outre au moins un autre agent antidiabétique.

## Patentansprüche

1. Verbindung der Formel (I) 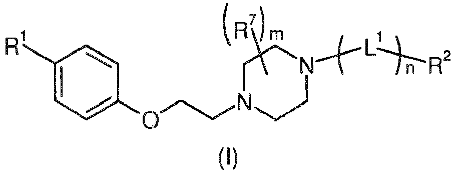 wobei:
▪ R¹:
- eine -C(O)CR³R⁴CR⁵R⁶C(O)OH Gruppe; oder
- eine 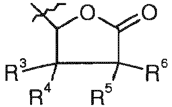 Gruppe darstellt;
▪ n 0 oder 1 darstellt;
▪ m 0 oder 1 darstellt;
▪ L¹ eine -C(O)-; -C(O)O- oder -S(O)₂- Gruppe darstellt;
▪ R²:
- eine Phenyl-Gruppe, nicht substituiert oder substituiert mit einem oder mehreren Substituenten, gleich oder verschieden, ausgewählt aus:
- einer C₁ bis C₆ Alkoxygruppe, linear oder verzweigt;
- einem Halogenatom;
- einer C₁ bis C₅ Kohlenwasserstoffgruppe, linear oder verzweigt, substituiert mit einem oder mehreren Halogenatomen;
- einer C₁ bis C₅ Kohlenwasserstoffgruppe, linear oder verzweigt;
- einer Cyanogruppe (-CN);
- einer Alkylsulfonylgruppe (-S(O)₂-Alkyl), wobei das Alkyl linear oder verzweigt ist, mit C₁ bis C₅;
- eine aromatische Heterozyklusgruppe mit 5, 6 oder 7 Gliedern, umfassend 1 oder 2 Heteroatome, gleich oder verschieden, ausgewählt aus Stickstoff, Schwefel und Sauerstoff;
- eine aromatische Polyheterozyklusgruppe mit 9 oder 10 Gliedern, umfassend 1, 2 oder 3 Heteroatome, gleich oder verschieden, ausgewählt aus Stickstoff, Schwefel und Sauerstoff;
- eine -L²-Phenyl-Gruppe;
- oder
- eine C₁ bis C₅ Kohlenwasserstoffgruppe, linear oder verzweigt, darstellt;
▪ L² ein C₁ bis C₅ Alkyl, linear oder verzweigt, darstellt;
▪ R³, R⁴, R⁵ und R⁶ ein Wasserstoffatom darstellen;
▪ R⁷
ein C₁ bis C₅ Alkyl, linear oder verzweigt, darstellt;
ihre Enantiomere, Diastereoisomere und ihre Salze der Addition mit pharmazeutisch annehmbaren Basen oder Säuren.

2. Verbindung gemäß Anspruch 1, wobei R²:
- ein Phenyl, unsubstituiert oder substituiert mit einem oder mehreren Substituenten, gleich oder verschieden, ausgewählt aus:
• einer C₁ bis C₆ Alkoxygruppe, linear oder verzweigt;
• einem Halogenatom;
• einer C₁ bis C₅ Alkylgruppe, linear oder verzweigt;
• einer C₁ bis C₅ Alkylgruppe, linear oder verzweigt, substituiert mit einem oder mehreren Halogenatomen;
• einer Cyanogruppe (-CN);
• einer Alkylsulfonylgruppe (-S(O)₂-Alkyl), wobei das Alkyl linear oder verzweigt ist, mit C₁ bis C₅; oder
- ein monocyclisches oder polycyclisches Heteroaryl ausgewählt aus 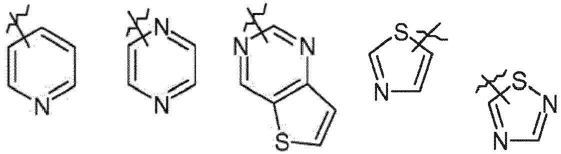
- eine Alk-Phenylgruppe, wobei Alk ein C₁ bis C₅ Alkyl, linear oder verzweigt, ist; oder
- eine C₁ bis C₅ Alkylgruppe, linear oder verzweigt darstellt.

3. Verbindung gemäß einem der Ansprüche 1 oder 2, wobei R¹ eine -C(O)CR³R⁴CR⁵R⁶C(O)OH Gruppe darstellt.

4. Verbindung gemäß einem der Ansprüche 1 bis 3, wobei n=0.

5. Verbindung gemäß einem der Ansprüche 1 bis 3, wobei n=1.

6. Verbindung gemäß einem der Ansprüche 1 bis 5, wobei m=0.

7. Verbindung gemäß einem der Ansprüche 1 bis 4, wobei:
R¹ eine -C(O)CR³R⁴CR⁵R⁶C(O)OH Gruppe darstellt;
R² eine Gruppe darstellt ausgewählt aus:
- einer Phenylgruppe, unsubstituiert oder substituiert mit einem oder mehreren Substituenten ausgewählt aus einer C₁ bis C₆ Alkoxygruppe, linear oder verzweigt; einem Halogenatom;
- einer aromatischen Heterozyklusgruppe mit 6 Gliedern umfassend 1 oder 2 Heteroatome, gleich oder verschieden, ausgewählt aus Stickstoff, Schwefel und Sauerstoff.

8. Verfahren zur Herstellung (P1) einer Verbindung gemäß einem der Ansprüche 1 bis 7, umfassend:
(a) die Schützung der Säurefunktion einer Verbindung der Formel (II) 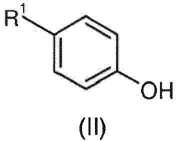 wobei R¹ -C(O)CR³R⁴CR⁵R⁶C(O)OH darstellt; R³, R⁴, R⁵ und R⁶ wie in den Ansprüchen 1 bis 7 definiert sind
(b) die Reaktion der Verbindung erhalten in Schritt (a) mit einer Verbindung der Formel R⁹-(CH₂)₂-R⁸, wobei R⁸ und R⁹, gleich oder verschieden, eine Abgangsgruppe darstellen, vorzugsweise ist R⁹ ein besseres Nukleofug als R⁸;
(c) die Reaktion der Verbindung erhalten in Schritt (b) mit einer Verbindung der Formel (III) 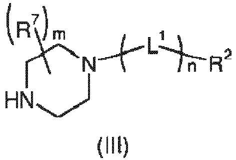 wobei L¹, n, m, R² und R⁷ wie in den Ansprüchen 1 bis 7 definiert sind;
(d) Entschützung der Verbindung erhalten in Schritt (c).

9. Verfahren zur Herstellung (P2) einer Verbindung gemäß einem der Ansprüche 1 bis 7, umfassend:
(i) die Reaktion einer Verbindung der Formel (III) 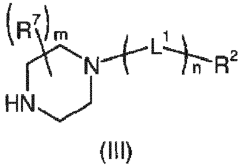 wobei L¹, m, n, R² und R⁷ wie in den Ansprüchen 1 bis 7 definiert sind, mit einer Verbindung der Formel R⁹-(CH₂)₂-R⁸, wobei R⁸ und R⁹ wie in Anspruch 8 definiert sind;
(ii) die Schützung der Säurefunktion einer Verbindung der Formel (II) 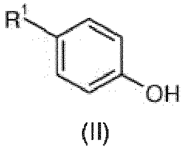 wobei R¹ -C(O)CR³R⁴CR⁵R⁶C(O)OH darstellt; R³, R⁴, R⁵ und R⁶ wie in den Ansprüchen 1 bis 7 definiert sind,
(iii) die Reaktion zwischen der Verbindung erhalten in Schritt (i) und der Verbindung erhalten in Schritt (ii);
(iv) die Entschützung der Verbindung erhalten in Schritt (iii).

10. Verbindung gemäß einem der Ansprüche 1 bis 7 als Medikament.

11. Verbindung gemäß einem der Ansprüche 1 bis 7 für ihre Verwendung zur Behandlung von Erkrankungen, die mit dem Syndrom der Insulinresistenz assoziiert sind.

12. Verbindung für ihre Verwendung gemäß Anspruch 11 zur Behandlung des Diabetes Typ (II).

13. Pharmazeutische Zusammensetzung umfassend als Wirksubstanz mindestens eine Verbindung gemäß einem der Ansprüche 1 bis 7.

14. Pharmazeutische Zusammensetzung gemäß Anspruch 13, umfassend des Weiteren mindestens einen anderen antidiabetischen Wirkstoff.

## Claims

1. Compound of formula (I) 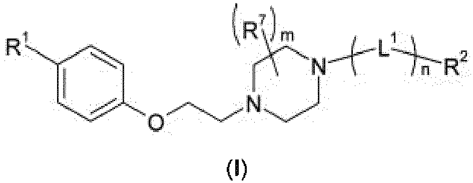 in which:
▪ R¹ represents:
- a -C(O)CR³R⁴CR⁵R⁶C(O)OH group; or
- a group 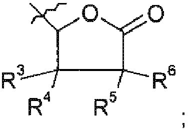
▪ n represents 0 or 1;
▪ m represents 0 or 1;
▪ L¹ represents a -C(O)-; -C(O)O- or -S(O)₂- group;
▪ R² represents:
- a phenyl group non-substituted or substituted by one or more substituants, identical or different chosen among:
- a C₁ to C₆ alkoxy group, linear or branched;
- a halogen atom;
- a C₁ to C₅ linear or branched hydrocarbon group, substituted by one or more halogen atoms;
- a C₁ to C₅ linear or branched hydrocarbon group;
- a cyano group (-CN); or
- a sulfonylalkyl group (-S(O)₂-alkyl), in which the alkyl is linear or branched, C₁ to C₅;
- a heterocycle aromatic group with 5, 6 or 7 members comprising 1 or 2 heteroatoms, identical or different chosen among nitrogen, sulfur and oxygen;
- a polyheterocycle aromatic group with 9 to 10 members, comprising 1, 2 or 3 heteroatoms identical or different chosen among nitrogen, sulfur and oxygen ;
- a -L²-phenyl group; or
- a hydrocarbon group, linear or branched, C₁ to C₅;
▪ L² represents an alkyl, linear or branched, C₁ to C₅;
▪ R³, R⁴, R⁵ and ^{R6}, represent a hydrogen atom;
▪ R⁷ represent an alkyl, linear or branched, C₁ to C₅;
enantiomers and diastereoisomers thereof and the addition salts thereof with pharmaceutically acceptable bases or acids.

2. Compound according to claim 1, in which R² represents
- a phenyl, non-substituted or substituted by one or more substituents, identical or different, chosen from:
• a C₁ to C₆ alkoxy group, linear or branched;
• a halogen atom;
• a C₁ to C₅ alkyl group, linear or branched;
• a C₁ to C₅ alkyl group, linear or branched, substituted by one or more halogen atoms;
• a cyano group (-CN);
• a sulfonylalkyl group (-S(O)₂-alkyl), in which the alkyl is linear or branched, C₁ to C₅; or
- monocyclic or polycyclic heteroaryl chosen among: 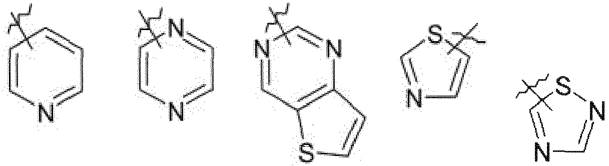
- an -Alk-phenyl, Alk being a C₁ to C₅ alkyl group, linear or branched; or
- a C₁ to C₅ alkyl group, linear or branched.

3. Compound according to claims 1 or 2 wherein R¹ represents a group C(O)CR³R⁴CR⁵R⁶C(O)OH.

4. Compound according to anyone of claims 1 to 3 wherein n=0.

5. Compound according to anyone of claims 1 to 3 wherein n=1.

6. Compound according to anyone of claims 1 to 5, wherein m=0.

7. Compound according to anyone of claims 1 to 4, wherein:
R¹ represents a group C(O)CR³R⁴CR⁵R⁶C(O)OH;
R² represents a group chosen among:
- A phenyl group, non-substituted or substituted by one or more substituents chosen among: an alkoxy group in C1-C6, linear or branched; an halogen atom;
- An aromatic heterocycle group with 6 members comprising 1 or 2 heteroatoms, identical or different, chosen among nitrogen, sulfur or oxygen.

8. Method (P1) for preparing a compound according to any one of claims 1 to 7, comprising:
(a) the protection of the acid function of a compound of formula (II) 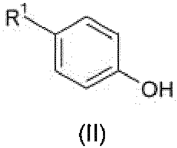 in which R¹ represents -C(O)CR³R⁴CR⁵R⁶C(O)OH; R³, R⁴, R⁵ and R⁶ are as defined by claims 1 and 7;
(b) the reaction of the compound obtained at step (a) with a compound of formula R⁹-(CH₂)₂-R⁸ in which R⁸ and R⁹, identical or different, represent a leaving group, and preferably R⁹ is a better nucleofuge than R⁸;
(c) the reaction of the compound obtained at step (b) with a compound of formula (III) 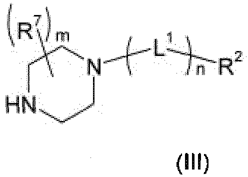 in which L¹, n, m, R² and R⁷ are as defined by claims 1 to 7;
(d) deprotection of the compound obtained at step (c).

9. Method (P2) for preparing a compound according to any one of claims 1 to 7, comprising:
(i) the reaction of a compound of formula (III) 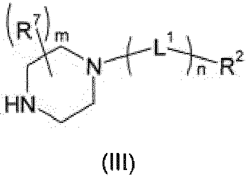 in which L¹, n, m, R² and R⁷ are as defined by claims 1 to 7 with a compound of formula R⁹-(CH₂)₂-R⁸, in which R⁸ and R⁹ are as defined in claim 8;
(ii) the protection of the acid function of a compound of formula (II) 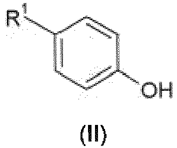 in which R¹ represents -C(O)CR³R⁴CR⁵R⁶C(O)OH; R³, R⁴, R⁵ and R⁶ are as defined in claims 1 to 7;
(iii) the reaction between the compound obtained at step (i) and the compound obtained at step (ii);
(iv) deprotection of the compound obtained at step (iii).

10. Compound according to any one of claims 1 to 7 as a medication.

11. Compound according to any one of claims 1 to 7 for use thereof for treating pathologies associated with the insulin-resistance syndrome.

12. Compound for use thereof according to claim 11 for treating type 2 diabetes.

13. Pharmaceutical composition comprising, as active principle, at least one compound according to any one of claims 1 to 7.

14. Pharmaceutical composition according to claim 13, also comprising at least one other anti-diabetic agent.
